# EUROPÄISCHE PATENTSCHRIFT

(11) **EP 4 360 558 B1**
(45) Veröffentlichungstag und Bekanntmachung des Hinweises auf die Patenterteilung: **15.04.2026**
(21) Anmeldenummer: 22204679.9
(22) Anmeldetag: 31.10.2022
(51) Int. Cl.: A61B 6/03, A61B 6/00

(54) **VERFAHREN ZUR BEREITSTELLUNG EINES VIRTUELLEN, NICHT-KONTRASTIERTEN BILDDATENSATZES**
METHOD FOR PROVIDING A VIRTUAL, NON-CONTRAST IMAGE DATA SET
PROCÉDÉ DE FOURNITURE D'UN ENSEMBLE DE DONNÉES D'IMAGE VIRTUELLE NON CONTRASTÉES

(43) Veröffentlichungstag der Anmeldung: 01.05.2024
(73) Patentinhaber: Siemens Healthineers AG, 91301 Forchheim (DE)
(72) Erfinder: Ditt, Hendrik, 91413 Neustadt an der Aisch (DE); Taubmann, Oliver, 91365 Weilersbach (DE)
(74) Vertreter: Siemens Healthineers Patent Attorneys

(56) Entgegenhaltungen:
- US-A1- 2014 343 358
- US-A1- 2017 265 829
- US-A1- 2022 087 631
- US-B1- 6 248 074
- US-B2- 11 883 222
- SHI-FENG TIAN ET AL: "Virtual Non-Contrast Computer Tomography (CT) with Spectral CT as an Alternative to Conventional Unenhanced CT in the Assessment of Gastric Cancer", ASIAN PACIFIC JOURNAL OF CANCER PREVENTION, vol. 16, no. 6, 3 April 2015 (2015-04-03), TH, pages 2521 - 2526, XP055454406, ISSN: 1513-7368, DOI: 10.7314/APJCP.2015.16.6.2521

## Beschreibung

Die Erfindung betrifft ein Verfahren zur Bereitstellung eines virtuellen, nicht-kontrastierten Bilddatensatzes eines Patienten basierend auf einem Mehrphasen CT-Angiographie-Bilddatensatz (mCTA-Bilddatensatz) des Patienten, welcher zumindest drei CT-Bilddatensätze umfasst, die einen Abbildungsbereich des Patienten zu drei verschiedenen Zeitpunkten relativ zu einer Kontrastmittelgabe abbilden. Weiterhin betrifft die Erfindung eine zugehörige Vorrichtung zur Bereitstellung eines virtuellen, nicht-kontrastierten Bilddatensatzes eines Patienten, ein Computertomographie-Gerät, ein Computerprogrammprodukt und ein computerlesbares Speichermedium.

Bei einem akuten Schlaganfall müssen mehrere Untersuchungen durchgeführt werden, um die Ursache und den Schweregrad des Schlaganfalls zu ermitteln. In der Regel wird zunächst eine Computertomographie (CT)-Untersuchung ohne Gabe von Kontrastmittel durchgeführt, um herauszufinden, ob im Patienten eine Blutung vorliegt, und um nach frühen Anzeichen für einen Schlaganfall zu suchen (z. B. Bestimmung hyperdenser Mediazeichen, des "Alberta Stroke Program Early CT Score", kurz ASPECTS, usw.). Wenn keine Blutung festgestellt werden kann, wird eine CT-Angiographie (CTA) durchgeführt, gefolgt von einem Perfusions-CT-Untersuchung (CTP). Bei der wird CTP wiederholt der interessierende Bereich über einen gewissen Zeitraum zu mehreren Zeitpunkten gescannt, beispielsweise im 1,5 Sekundentakt über eine Dauer von 40Sekunden und damit ein zeitaufgelöster 3D-Datensatz generiert, der die Anflutung und Abflutung des Kontrastmittels in den Gefäßen des interessierenden Bereichs, insbesondere des Gehirns, zeigt. In der CTA kann das Gerinnsel lokalisiert werden, während die CTP Aufschluss über den Schweregrad des Schlaganfalls geben und die Frage beantwortet kann, ob es noch einen Bereich überlebensfähiger Zellen (Penumbra) angrenzend an einer zentralen Nekrosezone gibt, der durch eine Therapie gerettet werden könnte. In einigen Krankenhäusern wird diese Perfusions-CT-Untersuchung durch eine Mehrphasen-CTA (mCTA) ersetzt. Bei einer mCTA-Akquisition werden nach der ersten CTA vom Aortenbogen bis zum Scheitelpunkt in der Regel lediglich zwei weitere Aufnahmen durchgeführt, bei denen nur das Gehirn gescannt wird. Diese drei Scans liefern eine deutlich geringere Zeitauflösung als ein CTP, aber mehr Informationen über die Verteilung von Kontrastmittel im Gehirn als eine reine CTA.

Es besteht die Möglichkeit, die mCTA-Daten quantitativ zu nutzen und daraus Ergebnisbilder zu erstellen, ähnlich wie sie bei der Analyse von CT-Perfusionsscans verwendet werden. Dies umfasst sogenannte "Perfusionskarten", z. B. CBF (cerebral blood flow, dt.: zerebraler Blutfluss), welche angibt, wie viel Volumen Blut (ml) pro Masse Gewebe (g) pro Zeit (min) fließt, CBV (cerebral blood volume, dt.: zerebrales Blutvolumen), welche angibt, wie viel Volumen Blut (ml) pro Masse Gewebe (g) vorzufinden ist oder TTP (Time-to-Peak, dt: "Zeit bis zum Maximalwert", auch Zeit bis zur maximalen Hyperdensität genannt), welche angibt, wie viel Zeit ein Kontrastmittel-Bolus benötigt, bis er sich in einer bestimmten Gewebe-Region maximal anreichert.

Das Dokument US20170265829A1 offenbart ein Verfahren zur farblichen Visualisierung der unterschiedlichen Phasen des Blutflusses in mCTA-Aufnahmen.

Das Dokument US20220087631A1 offenbart ein Verfahren von funktionalen Perfusionskarten auf Basis von mCTA-Aufnahmen.

Untersuchungen mittels mCTA beschränken sich nicht auf Schlaganfall-Patienten. Daneben kann es auch andere Anwendungen geben, beispielsweise für eine Tumorbeurteilung.

Für die Erstellung dieser Perfusionsbilddatensätze, also der Perfusionskarten, ist jedoch ein Basisbilddatensatz des diagnostisch relevanten Bereichs erforderlich, welches einem nicht-kontrastiertem CT-Bilddatensatz, d.h. einem CT-Bilddatensatz ohne das Vorliegen von Kontrastmittel, entspricht. Bei der CTP stellt dies in der Regel kein Problem dar, da hier der Scan bereits startet, wenn das Kontrastmittel den interessierenden Bereich noch nicht erreicht hat.

Zwar liegt häufig außerdem, wie zuvor beschrieben, ein CT-Bilddatensatz ohne eine vorherige Gabe von Kontrastmittel vor, im Folgenden auch nativer, nicht-kontrastierter CT-Bilddatensatz genannt, da dieser häufig am Beginn der Untersuchungen eines Schlaganfall-Patienten steht. Jedoch wird dieser in der Regel bei anderen Aufnahmeparametern aufgenommen wie die darauffolgenden mCTA-Aufnahmen. So wird dieser erste Scan, also der native, nicht-kontrastierte CT-Bilddatensatz, normalerweise mit einer höheren Röntgenröhrenspannung, beispielsweise mit 120 kVp, durchgeführt, um eine optimale Bildqualität für Weichgewebe zu erreichen. Im Gegensatz dazu werden CTA-Scans in der Regel mit einer dazu relativ geringeren Röntgenröhrenspannung, beispielsweise 80-100 kVp, durchgeführt, um einen besseren Kontrastmittelkontrast für die Visualisierung des Gefäßsystems zu erzielen. Weiterhin werden auf native, nicht-kontrastierte CT-Bilddatensätze häufig spezielle Bildfilter angewendet, wohingegen auf CTA-Bilddatensätze in der Regel andere Filter angewendet.

Trotz dieser Unterschiede wird derzeit in der Regel der native, nicht-kontrastierte Bilddatensatz als Basisbilddatensatz für Berechnung für Perfusionskarten auf Basis von mCTA-Bilddatensätzen genutzt. Dies führt jedoch aufgrund der unterschiedlichen Aufnahmeparametern zu Problemen und Artefakten.

Aufgabe der Erfindung ist es daher, ein Verfahren bereitzustellen, welches es erlaubt einen verbesserten nicht-kontrastierten Bilddatensatzes eines Patienten beispielsweise für eine optionale, spätere Verarbeitung des Mehrphasen CT-Angiographie-Bilddatensatz (mCTA-Bilddatensatz) des Patienten bereitzustellen. Ebenso ist es Aufgabe eine zughörige Vorrichtung, ein Computertomographie-Gerät, ein Computerprogrammprodukt und ein computerlesbares Speichermedium bereitzustellen.

Die Aufgabe wird gelöst durch das Verfahren und die Vorrichtungen, die in den unabhängigen Patentansprüchen beschrieben sind. Vorteilhafte und für sich gesehen erfinderische Ausgestaltungen sind Gegenstand der Unteransprüche und der nachfolgenden Beschreibung.

Die Erfindung betrifft ein Verfahren zur Bereitstellung eines virtuellen, nicht-kontrastierten CT-Bilddatensatzes eines Patienten.

Ein virtueller, nicht-kontrastierter CT-Bilddatensatz entspricht einem Bilddatensatz, welcher den Abbildungsbereich des Patienten ohne die Beeinflussung der Bildwerte durch ein Kontrastmittel abbildet. Der virtuelle, nicht-kontrastierte CT-Bilddatensatz wird als virtuell bezeichnet, im Gegensatz zum nativen, nicht-kontrastierten CT-Bilddatensatz, da dieser nicht auf der Akquisition von Messdaten basiert, welche ohne Kontrastmittelgabe erfasst wurden, sondern nachträglich mittels einer Recheneinheit basierend auf Messdaten erzeugt wird, welche mit Kontrastmittelgabe erfasst wurden.

Die im Rahmen des Verfahrens bereitgestellten und ermittelten CT-Bilddatensätze, umfassend den Minimumintensitätsbilddatensatz, können insbesondere jeweils ein dreidimensionaler Bilddatensatz (3D Bilddatensatz) sein. Ein 3D Bilddatensatz erlaubt eine dreidimensionale, insbesondere räumlich dreidimensionale, Darstellung des Abbildungsbereichs des Patienten. Ein 3D Bilddatensatz kann auch als eine Mehrzahl an Schichtbilddatensätzen dargestellt werden. Ein Schichtbilddatensatz umfasst jeweils eine Schicht des 3D Bilddatensatz an einer Position entlang einer ausgezeichneten Achse. Ein Schichtbilddatensatz erlaubt dann jeweils eine zweidimensionale, insbesondere räumlich zweidimensionale, Darstellung der jeweiligen Schicht.

Vorteilhafterweise umfasst ein solcher 3D Bilddatensatz mehrere Voxel, insbesondere Bildpunkte. Dabei kann jedes Voxel vorzugsweise jeweils einen Bildwert, insbesondere einen CT-Bildwert in HU ("Hounsfield Units") oder einen dazu analogen Intensitätswert, aufweisen. Analog dazu kann ein Schichtbilddatensatz mehrere Pixel, insbesondere Bildpunkte, umfassen. Dabei kann jeder Pixel vorzugsweise jeweils einen Bildwert, insbesondere einen CT-Bildwert in HU ("Hounsfield Units") oder eine dazu analogen Intensitätswert, aufweisen. Die zumindest drei CT-Bilddatensätze können sich insbesondere hinsichtlich ihrer Bildauflösung entsprechen.

Der Patient kann ein tierischer Patient und/oder ein menschlicher Patient sein. Ferner kann der Abbildungsbereich des Patienten einen anatomischen und/oder räumlichen Bereich des Patienten umfassen, der einen vorbestimmten Gewebebereich und/oder einen für eine Diagnose notwendigen räumlichen Bereich umfasst. Der Abbildungsbereich kann dabei einen Körperbereich, beispielsweise den Kopf oder den Thorax, umfassen.

Das Bereitstellen der zumindest drei CT-Bilddatensätze des mCTA-Bilddatensatzes kann beispielsweise ein Erfassen und/oder Auslesen eines computerlesbaren Datenspeichers und/oder ein Empfangen aus einer Speichereinheit umfassen. Ferner kann das erfindungsgemäße Verfahren auch eine Akquisition der zumindest drei CT-Bilddatensätze des mCTA-Bilddatensatzes mittels eines CT-Geräts umfassen, welche anschließend für die weiteren Schritte des Verfahrens bereitgestellt werden können. Das Ausgeben kann beispielsweise ein Ausgeben an eine nachgelagerte Verarbeitungseinheit umfassen, welche den bereitgestellten virtuellen, nicht-kontrastierten CT-Bilddatensatz weiterverarbeitet, beispielsweise Perfusionskarten erzeugt. Die Ausgabe kann auch eine Ausgabe an eine Darstellungseinheit, z.B. umfassend einen Monitor, umfassen, welcher die Darstellung des Bilddatensatzes für einen Anwender erlaubt.

Die zumindest drei CT-Bilddatensätze, ebenso wie der virtuelle, nicht-kontrastierte CT-Bilddatensatz und der Minimumintensitätsbilddatensatz bilden alle zumindest den Abbildungsbereich des Patienten ab. Der Abbildungsbereich umfasst insbesondere den Bereich es Patienten, welcher mittels der aus dem mCTA-Bilddatensatz gewonnen Bildinformation, in einer nachgelagerten Befundung bewertet werden soll. Jedoch können, müssen jedoch nicht, die Aufnahmebereiche der zumindest drei CT-Bilddatensätze, für welche Messdaten aufgenommen worden sind, unterschiedlich sein, sofern sie jeweils den Abbildungsbereich umfassen. So kann zum Beispiel der Aufnahmebereich des zeitlich ersten der zumindest CT-Bilddatensätze einen anderen, insbesondere ausgedehnteren, Aufnahmebereich umfassen als der zeitlich zweite und/oder dritte der zumindest drei CT-Bilddatensätze. Der Abbildungsbereich kann dem hinsichtlich der abgebildeten räumlichen Ausdehnung kleinsten der Aufnahmebereiche der zumindest drei CT-Bilddatensätze entsprechen. Er kann jedoch auch nur ein Ausschnitt davon sein.

Beispielsweise umfasst der Abbildungsbereich insbesondere das Gehirn eines Patienten. Vorteilhafterweise kann ein virtuelle, nicht-kontrastierte CT-Bilddatensatz für eine Weiterverarbeitung des mCTA-Bilddatensatzes, z.B**.** für Perfusionsbilddatensätze des Gehirns, bereitgestellt werden. So kann auf Basis eines solchen virtuellen, nicht-kontrastierten CT-Bilddatensatz eine verbesserte Schlaganfalldiagnostik ermöglich werden kann, wenn der virtuelle, nicht-kontrastierte CT-Bilddatensatz bei der Bereitstellung von Perfusionsbilddatensätzen zum Einsatz kommt. Beispielweise kann der Aufnahmebereich des ersten CT-Bilddatensatzes zumindest den Patienten vom Aortenbogen bis zum Scheitel umfassen, und der Aufnahmebereich des zweiten CT-Bilddatensatzes und/oder der Aufnahmebereich des dritten CT-Bilddatensatzes zumindest den Patienten von der Schädelbasis bis zum Scheitel abbilden. Beispielsweise entspricht der Abbildungsbereich dem Aufnahmebereich des zweiten und/oder dritten CT-Bilddatensatzes oder zumindest einen Ausschnitt davon. In anderen Ausführungsformen kann der Abbildungsbereich auch einen anderen Körperbereich des Patienten abbilden.

Bildgebende Untersuchungen unter Verwendung von Kontrastmittel, beispielsweise dem Patienten intravenös verabreichtes Kontrastmittel basierend auf Iod, werden durchgeführt, um eine Sichtbarkeit von Blutgefäßen, Organgewebe, tumorösen Strukturen oder Blutungen zu verbessern. Über den zeitlichen Verlauf der Kontrastmittelverteilung können weitere diagnostisch relevante Informationen erhalten werden. Die zumindest drei CT-Bilddatensätze bilden den Abbildungsbereich des Patienten zu drei verschiedenen Zeitpunkten relativ zu, d.h. insbesondere zeitlich nach, einer Kontrastmittelgabe ab. Die zumindest drei CT-Bilddatensätze bilden also jeweils den Zustand der Kontrastmittelverteilung im Patienten zu verschiedenen Zeitpunkten nach einer Kontrastmittelgabe ab.

Beispielsweise bildet der zeitlich erste der zumindest drei CT-Bilddatensätze des mCTA-Bilddatensatzes den Zustand der Kontrastmittelverteilung im Patienten zum Höhepunkt einer arteriellen Phase ab. Beispielsweise bildet der zeitlich zweite der zumindest drei CT-Bilddatensätze den Zustand der Kontrastmittelverteilung im Patienten zum Höhepunkt der venösen Phase ab. Beispielsweise bildet der zeitlich dritte der zumindest drei CT-Bilddatensätze den Zustand der Kontrastmittelverteilung im Patienten in einer spätvenösen Phase ab. Es können bei verschiedenen Anwendungen auch unterschiedliche Phasen gewählt werden.

Der jeweilige Zeitpunkt der Messdatenakquisition für die zumindest drei CT-Bilddatensätze kann auf Erfahrungswerten basieren oder beispielsweise zumindest teilweise über Bolus-Tracking- oder Testbolus-Methoden bestimmt sein.

Der relative Zeitabstand zwischen dem zeitlich ersten CT-Bilddatensatzes und dem zweiten CT-Bilddatensatzes der zumindest drei CT-Bilddatensätze bzw. der relative Zeitabstand zwischen dem zeitlich zweiten CT-Bilddatensatzes und dem dritten CT-Bilddatensatzes der zumindest drei CT-Bilddatensätze beträgt in bevorzugten Ausbildungen mindestens 5s beträgt. Beispielsweise startet die Datenaufnahme des zweiten CT-Bilddatensatzes 7, 8 oder 10s nach dem Start des ersten CT-Bilddatensatzes, beispielsweise startet die Datenaufnahme des dritten CT-Bilddatensatzes zwischen 7, 8 oder 10s nach dem Start des zweiten CT-Bilddatensatzes. Für die mCTA im Rahmen einer Diagnostik im Rahmen eines Schlaganfalls ist ein Zeitabstand von 7-10s besonders vorteilhaft. Für andere Anwendungen kann dies davon abweichen. Insbesondere wird die zeitliche Verzögerung zwischen den jeweiligen Starts der Datenaufnahme derart gewählt, dass die CT-Bilddatensätze für eine nachfolgende Diagnostik geeignet sind. Dabei kann sich der relative Zeitabstand zwischen dem zeitlich ersten CT-Bilddatensatzes und dem zweiten CT-Bilddatensatzes bzw. der relative Zeitabstand zwischen dem zeitlich zweiten CT-Bilddatensatzes und dem dritten CT-Bilddatensatzes der zumindest drei CT-Bilddatensätze verschieden sein.

Der Minimumintensitätsbilddatensatz bildet zumindest den Abbildungsbereich ab. Er kann also hinsichtlich der Abbildung dem hinsichtlich der räumlichen Ausdehnung kleinsten der Aufnahmebereiche der zumindest drei CT-Bilddatensätze entsprechen. Er kann jedoch auch nur ein Ausschnitt davon abbilden.

Die Bildwerte des Minimumintensitätsbilddatensatz basieren jeweils auf dem minimalen Wert unter den Bildwerten der im Abbildungsbereich örtlich korrespondierenden Bildpunkte in den zumindest drei CT-Bilddatensätzen. Das heißt, der Bildwert eines Bildpunkts des Minimumintensitätsbilddatensatzes basiert jeweils auf dem Bildwert des örtlich korrespondierenden Bildpunktes im Abbildungsbereich desjenigen der zumindest drei CT-Bilddatensätze, welcher den minimalen Bildwert aufweist. Insbesondere können sie diesem entsprechen. Sie können jedoch auch jeweils auf diesem minimalen Wert basieren und angepasst sein. Für das Bilden des Minimumintensitätsbilddatensatzes kann beispielsweise ein vorläufiger Bilddatensatz gebildet werden, dessen Bildpunkte, d.h. Voxel bzw. Pixel, mit dem jeweiligen Bildwert eines der zumindest drei CT-Bilddatensätze versehen werden, welcher minimal ist. Örtlich korrespondieren bedeutet dabei, dass örtlich korrespondierende Bildpunkte in den zumindest drei CT-Bilddatensätzen und entsprechen auch im Minimumintensitätsbilddatensatz jeweils den gleichen Bildbereich des Patienten abbilden. Dieses Vorgehen kann also als eine Minimumintensitätsprojektion basierend auf dem mCTA-Bilddatensatz entlang der Zeitachse verstanden werden. Der minimale Wert in einem CT-Bilddatensatz entspricht erfindungsgemäß aufgrund der üblichen Darstellung eines CT-Bilddatensatzes demjenigen Wert, welcher der höchsten Transmission an Röntgenstrahlung entspricht, d.h. der geringsten Absorption.

Die Überlegung hinter dem erfindungsgemäßen Vorgehen stellt sich entsprechend wie folgt dar: Da die zumindest drei CT-Bilddatensätze den Abbildungsbereich jeweils zu unterschiedlichen Zeitpunkten relativ zu der Kontrastmittelgabe darstellen, ist die Annahme, dass für jeden abgebildeten Bereich bzw. Gewebetyp im Abbildungsbereich eine Abbildung in einer der drei CT-Bilddatensätze vorhanden ist, in welcher dieser nicht von Kontrastmittel beeinflusst ist, so dass der Bildbereich desjenigen CT-Bilddatensatzes, für welchen das zutrifft für diesen Bereich als nicht-kontrastiertes Bild genutzt werden kann. Es kann dabei dann weiter ausgegangen werden, dass der jeweilige minimale Bildwert für einen korrespondierenden Bildpunkt bzw. Voxel in den zumindest drei CT-Bilddatensätze, demjenigen entspricht, der nicht von Kontrastmittel beeinflusst ist, da das Kontrastmittel zu einer Erhöhung des Bildwertes relativ zu einem Ausgangswert führen würde.

Übertragen auf das Beispiel eines Schlaganfallpatienten bedeutet dies: In gesundem Gehirngewebe erreicht das Kontrastmittel das Gewebe und Gefäße sehr früh, so dass der zeitlich erste der zumindest drei CT-Bilddatensätze eine Beeinflussung durch Kontrastmittel im Parenchym des Gehirns aufweist. Jedoch wird in diesen Teilen des Gehirns im zeitlich letzten der zumindest drei CT-Bilddatensätze kein Kontrastmittel mehr vorliegen, da dieses bereits ausgewaschen sein wird. Die Penumbra um den Kernbereich des Infarkts wird verzögert relativ zu gesundem Gewebe mittels des Kontrastmittels in Bilddatensätzen verstärkt sein. Diese wird folglich im zeitlich ersten der zumindest drei CT-Bilddatensätze unbeeinflusst vorliegen. Im betroffenen Gehirngewebe des Kernbereichs des Infarkts wird stets unbeeinträchtigt von Kontrastmittel abgebildet sein, da dieses dorthin nicht gelangt. Folglich liegt für jeden Gewebebereich jeweils zumindest eine Abbildung vor, welche nicht von Kontrastmittel beeinflusst ist, auf welcher ein virtueller, nicht-kontrastierter CT-Bilddatensatz basieren kann, der dann beispielweise als Basisbild für eine optionale Weiterverarbeitung des mCTA-Bilddatensatzes bereitgestellt werden kann.

Vorteilhaft kann mittels des erfindungsgemäßen Verfahrens ein nicht-kontrastierter CT-Bilddatensatz bereitgestellt werden, welcher auf Messdaten basiert, welche den Aufnahmebedingungen entsprechen, welche für die zumindest drei CT-Bilddatensätze angewendet wurden. Vorteilhaft kann eine Diagnostik und Weiterverarbeitung der Bilddatensätze basierend auf dem verbesserten nicht-kontrastierten Bilddatensatz erfolgen und damit eine verbesserte Qualität erreicht werden. Vorteilhaft kann ggf. auf eine weitere Dosisapplikation verzichtet werden, um einen zu dem mCTA-Bilddatensatz korrespondierenden nicht-kontrastierten CT-Bilddatensatz vom Patienten zu erzeugen, welcher auf Messdaten bei gleichen Aufnahmeparametern wie der mCTA-Bilddatensatz basiert.

Gemäß einer vorteilhaften Ausführungsvariante umfasst das Verfahren außerdem den Schritt:
- Durchführen einer Bewegungskorrektur der zumindest drei CT-Bilddatensätze, wodurch bewegungskorrigierte CT-Bilddatensätze bereitgestellt werden,
   und
   wobei der Minimumintensitätsbilddatensatz basierend auf den bewegungskorrigierten CT-Bilddatensätzen gebildet wird.

Das vorgeschlagene Verfahren kann insbesondere dann besonders vorteilhaft angewendet werden, wenn die zumindest drei CT-Bilddatensätze den Patienten bzw. den Abbildungsbereich in einer unveränderten Lage und unveränderten Zustand (abgesehen von der Kontrastmittelverteilung) abbilden. Aufgrund von Bewegungen der abgebildeten Strukturen zwischen den einzelnen Aufnahmen kann es zu störenden Bewegungsartefakten im Minimumintensitätsbilddatensatz kommen. Eine Folge dieser Bewegungen kann sein, dass der resultierende virtuelle, nicht-kontrastierte CT-Bilddatensatz nicht für eine folgende Weiterverarbeitung und darauf basierende Diagnose verwendet werden kann. Vorteilhaft kann eine Bewegungskorrektur Bewegungen des Patienten zwischen der Messdatenaufnahme für die zumindest drei CT-Bilddatensätze Rechnung tragen und deren Auswirkung verringern und so ein verbesserter Minimumintensitätsbilddatensatz bereitgestellt werden.

Für eine Bewegungskorrektur, wie sie im Rahmen des Verfahrens eingesetzt werden kann, gibt es verschiedene Möglichkeiten, welche dem Fachmann aus dem Stand der Technik bekannt sind. Ziel ist insbesondere eine Registrierung und Transformierung der Strukturen der zumindest drei CT-Bilddatensätze aufeinander, so dass in einer vorteilhaften Weise ein Minimumintensitätsbilddatensatz basierend auf den korrigierten zumindest drei CT-Bilddatensätze berechnet werden kann. Insbesondere kann eine bekannte rigide Registrierung genutzt werden. Es kann beispielsweise auch ein Verfahren wie in Zhang L, Chefd'hotel C, Bousquet G "Group-wise motion correction of brain perfusion images," (2010 IEEE International Symposium on Biomedical Imaging: From Nano to Macro, 2010, pp. 832-835, doi: 10.1109/ISBI.2010.5490115.) beschrieben genutzt werden. Es können jedoch auch andere Methoden zum Einsatz kommen, welche dazu führen, dass durch Bewegung des Patienten verursachte Artefakte im Minimumintensitätsbilddatensatz zumindest reduziert werden.

Gemäß einer vorteilhaften Ausbildungsvariante des Verfahrens sind außerdem folgende Schritte umfasst:
- Bereitstellen eines nativen, nicht-kontrastierten CT-Bilddatensatzes, welcher den Abbildungsbereich des Patienten ohne Kontrastmittelgabe abbildet, und
- Korrigieren des Minimumintensitätsbilddatensatz unter Einsatz des nativen, nicht-kontrastierten CT-Bilddatensatzes, wodurch ein korrigierter Minimumintensitätsbilddatensatz bereitgestellt wird, und
wobei
der ausgegebene virtuelle, nicht-kontrastierte Bilddatensatz auf dem korrigierten Minimumintensitätsbilddatensatz basiert.

Der native, nicht-kontrastierte CT-Bilddatensatz ist insbesondere bei Aufnahmeparametern aufgenommen, welche sich von den Aufnahmeparametern der zumindest drei CT-Bilddatensätze des Mehrphasen CT-Angiographie-Bilddatensatzes unterscheiden. Dies betrifft insbesondere Betriebsparameter der Röntgenquelle, wie eine Röntgenröhrenspannung und/oder ein Röntgenröhrenstrom. Der native, nicht-kontrastierte CT-Bilddatensatz kann beispielsweise bei einer höheren Röntgenröhrenspannung durchgeführt worden sein, als die zumindest drei CT-Bilddatensätze, um eine optimale Bildqualität für Weichgewebe zu erreichen. Der native, nicht-kontrastierte CT-Bilddatensatz wird in der Regel zeitlich vor den zumindest drei CT-Bilddatensätzen aufgenommen und bildet den Abbildungsbereich des Patienten vor der Kontrastmittelgabe und ohne Einfluss durch diese ab.

Wie bereits zuvor beschrieben, ist der direkte Einsatz des nativen, nicht-kontrastierten CT-Bilddatensatz aufgrund unterschiedlicher Aufnahmeparameter nur beschränkt für eine Weiterverarbeitung des mCTA-Bilddatensatzes beispielsweise zu Perfusionskarten geeignet. Jedoch kann dieser vorteilhaft eingesetzt werden, den Minimumintensitätsbilddatensatz zu korrigieren, um einen verbesserten virtuellen, nicht-kontrastierten CT-Bilddatensatz bereitzustellen. So können beispielsweise verrauschte Regionen in den zumindest drei CT-Bilddatensätzen des mCTA-Bilddatensatzes aufgrund der Berechnung des Minimums bei der Erstellung des Minimumintensitätsbilddatensatzes eine Verzerrung in Richtung einer lokalen Unterschätzung der Gewebedichte verursachen. Solche unterschätzten Regionen im virtuellen, nicht-kontrastierten CT-Bilddatensatz können (fälschlicherweise) in eine lokal höhere Kontrastmittelaufnahme resultieren bei einer Weiterverarbeitung der Bilddatensätze zu Perfusionskarten auf Basis des virtuellen, nicht-kontrastierten CT-Bilddatensatzes. Vorteilhaft kann basierend auf einem Vergleich des nativen, nicht-kontrastierten CT-Bilddatensatzes und Minimumintensitätsbilddatensatz eine Korrektur durchgeführt werden. Beispielsweise kann ein Vergleich eines Kontrastes von vorliegenden Strukturen oder Bildbereichen zu umliegenden Strukturen oder Bereichen benutzt werden, um eine Korrektur darauf basierend durchzuführen. Dies kann insbesondere dann erfolgen, wenn der Vergleich der Verhältnisse im nativen, nicht-kontrastierten CT-Bilddatensatz und im Minimumintensitätsbilddatensatz eine unerwartet große Abweichung aufweist. Ist ein Verhältnis bzw. ein Kontrast in beiden Bilddatensätzen ähnlich bzw. innerhalb eines vorgegebenen Erwartungsbereichs, so ist die Wahrscheinlichkeit größer, dass hier kein Artefakt vorliegt, sondern die Bildwerte tatsächlich so vorliegen und die Strukturen korrekt wiedergegeben sind.

Auch in diesem Fall kann vor einem Vergleich eine Bewegungskorrektur durchgeführt werden, um mögliche Bewegungen des Patienten zwischen der Akquisition des nativen, nicht-kontrastierten CT-Bilddatensatzes und der dem Minimumintensitätsbilddatensatz zu Grunde liegenden zumindest drei CT-Bilddatensätze Rechnung zu tragen.

In vorteilhaften Ausbildungen dieser Verfahrensvariante umfasst der Schritt des Korrigierens, dass für die Korrektur zuvor Bildbereiche im Minimumintensitätsbilddatensatz bestimmt werden, welche einen bestimmten Rauschwert überschreiten oder welche einen bestimmten Intensitätswert unterschreiten, und basierend auf einem Vergleich dieser bestimmten Bildbereiche mit örtlich korrespondierenden Bildbereichen in dem nativen, nicht-kontrastierten CT-Bilddatensatz zumindest ein Korrekturwert für die bestimmten Bildbereiche im Minimumintensitätsbilddatensatz bestimmt wird.

Ebenso ist es möglich, dass für die Korrektur Bildbereiche in zumindest einem der zumindest drei CT-Bilddatensätze bestimmt werden, welche einen bestimmten Rauschwert überschreiten oder welche einen bestimmten Intensitätswert unterschreiten, und basierend auf einem Vergleich zwischen jeweils dazu örtlich korrespondierenden Bildbereichen im Minimumintensitätsbilddatensatz und im nativen, nicht-kontrastierten CT-Bilddatensatz zumindest ein Korrekturwert für die bestimmten Bildbereiche im Minimumintensitätsbilddatensatz bestimmt wird.

Basierend auf dem Rauschwert oder einem geringen Intensitätswert können Bildbereiche bestimmt werden, welche mit höherer Wahrscheinlichkeit zu einer verzerrten Berechnung des Minimumintensitätsbilddatensatz führen. Vorteilhaft kann nach einer Identifizierung dieser Bereiche mit einer erhöhten Wahrscheinlichkeit eine verzerrte Bildwertbestimmung für den Minimumintensitätsbilddatensatz eine Prüfung und ggf. Korrektur erfolgen.

Alternativ zu einer Bestimmung von Bildbereichen basierend auf dem Minimumintensitätsbilddatensatz oder zumindest einem der zumindest drei CT-Bilddatensätze, kann das Korrigieren auch umfassen, womöglich zu korrigierende Bildbereiche in einem basierend auf dem unkorrigierten Minimumintensitätsbilddatensatz erstellten Perfusionsbilddatensatz zu bestimmen. Diese Verfahrensvariante umfasst dann, basierend auf den zumindest drei CT-Bilddatensätzen des Mehrphasen CT-Angiographie-Bilddatensatzes und dem unkorrigiert vorliegenden Minimumintensitätsbilddatensatz des Patienten einen Perfusionsbilddatensatz zu erstellen, Bildbereiche des Perfusionsbilddatensatzes mit lokal erhöhter Kontrastmittelaufnahme zu identifizieren, und basierend auf einem Vergleich zwischen dazu örtlich korrespondierender Bildbereiche im Minimumintensitätsbilddatensatz und örtlich korrespondierender Bildbereichen in dem nativen, nicht-kontrastierten CT-Bilddatensatz zumindest einen Korrekturwert zu bestimmen.

Wie zuvor beschrieben, können verrauschte Regionen in den zumindest drei CT-Bilddatensätzen des mCTA-Bilddatensatzes aufgrund der Berechnung des Minimums bei der Erstellung des Minimumintensitätsbilddatensatzes eine Verzerrung in Richtung einer lokalen Unterschätzung der Gewebedichte verursachen. Solche unterschätzten Regionen im vorläufigen unkorrigierten Minimumintensitätsbilddatensatz können (fälschlicherweise) in eine lokal höhere Kontrastmittelaufnahme resultieren bei einer Weiterverarbeitung der Bilddatensätze zu Perfusionskarten. Bereiche mit lokal höhere Kontrastmittelaufnahme in einem Perfusionsbilddatensatz stellen also Bereiche dar, in welchen mit erhöhter Wahrscheinlichkeit eine Falschbestimmung der Werte im Minimumintensitätsbilddatensatz vorliegen könnte. Diese werden identifiziert und einer Prüfung und ggf. Korrektur basierend auf einem Vergleich mit dem native, nicht-kontrastierte CT-Bilddatensatz unterzogen.

In den zuvor beschriebenen Varianten kann der Korrekturwert insbesondere auf einem Verhältnis der Bildwerte in den bestimmten Bildbereichen des Minimumintensitätsbilddatensatz bzw. des nativen, nicht-kontrastierten CT-Bilddatensatzes zu Bildwerten in einer Umgebung der bestimmten Bildbereiche des Minimumintensitätsbilddatensatz bzw. des nativen, nicht-kontrastierten CT-Bilddatensatzes basieren. Der Vergleich kann insbesondere umfassen, einen Kontrastwert zwischen vorliegenden Strukturen oder Bildbereichen zu umliegenden Strukturen oder Bereichen zu vergleichen. Ist ein Verhältnis in beiden Bilddatensätzen ähnlich oder innerhalb eines Erwartungsbereichs, so ist die Wahrscheinlichkeit größer, dass hier kein Artefakt, sondern die Bildwerte tatsächlich so vorliegen. Unterscheidet sich das Verhältnis signifikant oder liegt außerhalb der Erwartungen, so werden die Bildwerte in den zuvor bestimmten Bildbereichen des Minimumintensitätsbilddatensatzes mittels zumindest eines Korrekturwerts angepasst, beispielsweise in einer Art, so dass daraus ein Verhältnis ähnlich oder gleich wie im nativen, nicht-kontrastierten CT-Bilddatensatz resultiert oder innerhalb des Erwartungsbereichs liegt. Eine Justierung, ab wann eine Anpassung erfolgen sollte, beispielweise eines Erwartungsbereichs, und in welchem Maße eine Anpassung erfolgen muss, kann beispielweise anhand von Erfahrungswerten oder Messreihen erfolgen. Beispielweise kann auch die Vermessung von Phantomen dazu verwendet werden.

Die Erfindung betrifft außerdem ein Verfahren zur Bereitstellung eines Perfusionsbilddatensatzes, wobei ein Mehrphasen CT-Angiographie-Bilddatensatz des Patienten umfassend zumindest drei CT-Bilddatensätze, welche einen Abbildungsbereich des Patienten zu drei verschiedenen Zeitpunkten relativ zu einer Kontrastmittelgabe abbilden, bereitgestellt wird.

Insbesondere können basierend auf dem mCTA-Bilddatensatz und dem vorteilhaft erstellten virtuelle, nicht-kontrastierte CT-Bilddatensatz sogenannte "Perfusionskarten", z. B. CBF (cerebral blood flow, dt.: zerebraler Blutfluss), welche angibt, wie viel Volumen Blut (ml) pro Masse Gewebe (g) pro Zeit (min) fließt, oder CBV (cerebral blood volume, dt.: zerebrales Blutvolumen), welche angibt, wie viel Volumen Blut (ml) pro Masse Gewebe (g) vorzufinden ist, bereitgestellt werden und/oder darauf basierend weitere Parameter abgeleitet werden. Methoden zur Berechnung der Parameter sind dem Fachmann aus dem Stand der Technik bekannt und können in gleicher Weise auch hier angewendet werden. Vorteilhaft kann jedoch mittels des virtuelle, nicht-kontrastierte CT-Bilddatensatz ein verbesserter Perfusionsbilddatensatz bereitgestellt werden, welcher insbesondere weniger artefaktbehaftet sein kann. Damit können vorteilhaft Bilddatensätze und Werte mit hoher Qualität bereitgestellt werden, welche eine nachfolgenden Diagnose verbessert ermöglicht.

Die Erfindung betrifft weiterhin eine Vorrichtung zur Bereitstellung eines virtuellen, nicht-kontrastierten Bilddatensatzes eines Patienten.

Eine solche Vorrichtung zur Bereitstellung eines virtuellen, nicht-kontrastierten Bilddatensatzes eines Patienten kann insbesondere dazu ausgebildet sein, die zuvor beschriebenen erfindungsgemäßen Verfahren zur Bereitstellung eines virtuellen, nicht-kontrastierten Bilddatensatzes eines Patienten und ihre Aspekte auszuführen. Die Vorrichtung kann dazu ausgebildet sein, die Verfahren und ihre Aspekte auszuführen, indem die Schnittstellen und die Recheneinheit ausgebildet sind, die entsprechenden Verfahrensschritte auszuführen.

Die Vorrichtung kann weiterhin ausgebildet sein, basierend auf einem bereitgestellten Mehrphasen CT-Angiographie-Bilddatensatz des Patienten umfassend zumindest drei CT-Bilddatensätze, welche einen Abbildungsbereich des Patienten zu drei verschiedenen Zeitpunkten relativ zu einer Kontrastmittelgabe abbilden, und basierend auf einem nach einem der vorangehenden Ansprüchen basierend auf den zumindest drei CT-Bilddatensätzen bereitgestellten virtuellen, nicht-kontrastierten CT-Bilddatensatz des Abbildungsbereichs einen Perfusionsbilddatensatz zu erzeugen und bereitzustellen.

Bei der Vorrichtung bzw. der Recheneinheit kann es sich insbesondere um einen Computer, einen Mikrocontroller oder um einen integrierten Schaltkreis handeln. Alternativ kann es sich dabei um einen realen oder virtuellen Verbund von Computern handeln (ein englischer Fachbegriff für einen realen Verbund ist "Cluster", ein englischer Fachbegriff für einen virtuellen Verbund ist "Cloud"). Die Vorrichtung kann auch als virtuelles System ausgebildet sein, das auf einem realen Computer oder einem realen oder virtuellen Verbund von Computern ausgeführt wird (engl. virtualization).

Bei einer Schnittstelle kann es sich um eine Hardware- oder Softwareschnittstelle handeln (beispielsweise PCI-Bus, USB oder Firewire). Eine Recheneinheit kann Hardware-Elemente oder Software-Elemente aufweisen, beispielsweise einen Mikroprozessor oder ein sogenanntes FPGA (englisches Akronym für "Field Programmable Gate Array").

Die Schnittstellen können insbesondere mehrere Unterschnittstellen umfassen. Mit anderen Worten können die Schnittstellen auch eine Vielzahl von Schnittstellen umfassen. Die Recheneinheit können insbesondere mehrere Unterrecheneinheiten umfassen, die unterschiedliche Schritte der jeweiligen Verfahren ausführen. Mit anderen Worten kann die Recheneinheit auch als Vielzahl von Recheneinheiten aufgefasst werden.

Die Vorrichtung kann außerdem auch eine Speichereinheit umfassen. Eine Speichereinheit kann als nicht dauerhafte Arbeitsspeicher (Random Access Memory, kurz RAM) oder als dauerhafter Massenspeicher (Festplatte, USB-Stick, SD-Karte, Solid State Disk) realisiert sein.

Die Vorteile der vorgeschlagenen Vorrichtung entsprechen im Wesentlichen den Vorteilen des vorgeschlagenen Verfahrens zur Bereitstellung eines virtuellen, nicht-kontrastierten Bilddatensatzes eines Patienten. Hierbei erwähnte Merkmale, Vorteile oder alternative Ausführungsformen können ebenso auch auf die Vorrichtung übertragen werden und umgekehrt.

Die Erfindung betrifft weiterhin ein Computertomographie-Gerät aufweisend eine Vorrichtung zur Bereitstellung eines virtuellen, nicht-kontrastierten Bilddatensatzes eines Patienten wie zuvor beschrieben. Computertomographie-Gerät umfasst zumindest eine Röntgenquelle und einen gegenüber dazu angeordneten Detektor, welche auf einer rotierbaren Gantry angeordnet sind. Das Computertomographie-Gerät ist ausgebildet eine Mehrzahl an Projektionsdatensätzen aus verschiedenen Projektionswinkeln bei einer relativen Rotationsbewegung zwischen einer Röntgenquelle und einem zwischen der Röntgenquelle und dem Detektor angeordneten Patienten zu erfassen. Basierend auf der Mehrzahl an Projektionsdatensätzen können jeweils CT-Bilddatensätze rekonstruiert und für eine Weiterverarbeitung bereitgestellt werden. Das Computertomographie-Gerät ist insbesondere ausgebildet die zumindest drei CT-Bilddatensätze des mCTA-Bilddatensatzes bereitzustellen, welche den Abbildungsbereich des Patienten zu drei verschiedenen Zeitpunkten relativ zu einer Kontrastmittelgabe abbilden.

Dabei ist das Computertomographie-Gerät vorteilhafterweise zur Ausführung einer Ausführungsform des vorgeschlagenen Verfahrens zur Bereitstellung eines virtuellen, nicht-kontrastierten Bilddatensatzes eines Patienten ausgebildet.

Die Vorteile der vorgeschlagenen Computertomographie-Geräts entsprechen im Wesentlichen den Vorteilen des vorgeschlagenen Verfahrens zur Bereitstellung eines virtuellen, nicht-kontrastierten Bilddatensatzes eines Patienten. Hierbei erwähnte Merkmale, Vorteile oder alternative Ausführungsformen können ebenso auch auf die Vorrichtung übertragen werden und umgekehrt.

Die Erfindung betrifft weiterhin ein Computerprogrammprodukt mit einem Computerprogramm, welches direkt in einen Speicher einer Vorrichtung zur Bereitstellung eines virtuellen, nicht-kontrastierten Bilddatensatzes eines Patienten ladbar ist, mit Programmabschnitten, um alle Schritte eines erfindungsgemäßen Verfahrens zur Bereitstellung eines virtuellen, nicht-kontrastierten Bilddatensatzes eines Patienten auszuführen, wenn die Programmabschnitte von der Vorrichtung ausgeführt werden.

Ein Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Vorrichtung die erfindungsgemäßen Verfahrensschritte ausführen kann. Die Vorrichtung muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in eine Recheneinheit der Vorrichtung geladen werden kann.

Die Erfindung betrifft weiterhin ein Computerlesbares Speichermedium, auf welchem von einer Vorrichtung zur Bereitstellung eines virtuellen, nicht-kontrastierten Bilddatensatzes eines Patienten lesbare und ausführbare Programmabschnitte gespeichert sind, um alle Schritte eines erfindungsgemäßen Verfahrens zur Bereitstellung eines virtuellen, nicht-kontrastierten Bilddatensatzes eines Patienten wie zuvor beschrieben auszuführen, wenn die Programmabschnitte von der Vorrichtung ausgeführt werden.

Beispiele für ein computerlesbares Speichermedium sind eine DVD, ein Magnetband, eine Festplatte oder ein USB-Stick, auf welchem elektronisch lesbare Steuerinformationen, insbesondere Software, gespeichert ist.

Eine weitgehend softwaremäßige Realisierung hat den Vorteil, dass auch schon bisher verwendete Vorrichtungen und Recheneinheiten auf einfache Weise durch ein Software-Update nachgerüstet werden können, um auf die erfindungsgemäße Weise zu arbeiten. Ein Computerprogrammprodukt kann neben dem Computerprogramm gegebenenfalls zusätzliche Bestandteile wie z. B**.** eine Dokumentation und/oder zusätzliche Komponenten, sowie Hardware-Komponenten, wie z.B**.** Hardware-Schlüssel (Dongles etc.) zur Nutzung der Software, umfassen.

Neben dem zuvor beschriebenen erfindungsgemäßen Verfahren zur Bereitstellung eines virtuellen, nicht-kontrastierten CT-Bilddatensatz ist es alternativ auch möglich, basierend auf dem Einsatz einer trainierten Funktion einen solchen virtuellen, nicht-kontrastierten CT-Bilddatensatz zu erzeugen. Entsprechend könnte auch eine Vorrichtung vorgesehen sein, welche dazu ausgebildet sein, ein solches im Folgenden beschriebenes Verfahren auszuführen. Die Vorrichtung kann dazu ausgebildet sein, die Verfahren und ihre Aspekte auszuführen, indem geeignete Schnittstellen und eine Recheneinheit bereitgestellt sind, die ausgebildet sind, die entsprechenden Verfahrensschritte auszuführen.

Ein solches Verfahren würde dann zumindest umfassen, dass
- ein Mehrphasen CT-Angiographie-Bilddatensatzes des Patienten umfassend zumindest drei CT-Bilddatensätze, welche einen Abbildungsbereich des Patienten zu drei verschiedenen Zeitpunkten relativ zu einer Kontrastmittelgabe abbilden mittels einer Schnittstelle bereitgestellt wird,
- ein virtueller, nicht-kontrastierter CT-Bilddatensatz durch Anwenden einer trainierten Funktion auf den mCTA-Bilddatensatz mittels einer Recheneinheit erzeugt wird, wobei zumindest ein Parameter der trainierten Funktion auf einem Vergleich zwischen einem virtuellen, nicht-kontrastierten Trainings-Bilddatensatz und einem nicht-kontrastierten Vergleichs-Bilddatensatz angepasst ist, wobei der erzeugte virtuelle, nicht-kontrastierte Trainings-Bilddatensatz und der nicht-kontrastierten Vergleichs-Bilddatensatz miteinander verknüpft ist, und
- der erzeugte virtuelle, nicht-kontrastierte CT-Bilddatensatz anschließend mittels einer weiteren Schnittstelle ausgegeben wird.

Eine Erläuterung, was von einem virtuellen, nicht-kontrastierten Trainings-Bilddatensatz und einem nicht-kontrastierten Vergleichs-Bilddatensatz umfasst sein kann, wird im Folgenden näher erläutert werden.

Eine trainierte Funktion kann bevorzugt mittels eines künstlichen Intelligenzsystems, d.h. durch ein Verfahren des maschinellen Lernens, realisiert sein. Unter einem künstlichen Intelligenzsystem kann man ein System für die künstliche Generierung von Wissen aus Erfahrung bezeichnen. Ein künstliches System lernt aus Beispielen in einer Trainingsphase und kann nach Beendigung der Trainingsphase verallgemeinern. Die Verwendung eines solchen Systems kann ein Erkennen von Mustern und Gesetzmäßigkeiten in den Trainingsdaten umfassen. Nach der Trainingsphase kann der optimierte, d.h**.** trainierte, Algorithmus beispielsweise basierend auf einem bisher unbekannten mCTA-Bilddatensatz einen virtuellen, nicht-kontrastierten CT-Bilddatensatz ableiten. Das künstliche Intelligenzsystem kann ein künstliches neuronales Netz oder auch auf einem anderen Verfahren des maschinellen Lernens basieren. Insbesondere kann mittels einer trainierten Funktion basierend auf einem künstlichen Intelligenzsystems nach der Trainingsphase ein Ermitteln des virtuellen, nicht kontrastierten CT-Bilddatensatz besonders verlässlich und zeiteffizient automatisiert ermöglicht werden.

Eine trainierte Funktion bildet insbesondere Eingabedaten auf Ausgabedaten ab. Hierbei können die Ausgabedaten insbesondere weiterhin von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen oder der mehreren Parameter der trainierten Funktion kann insbesondere auf einem Paar aus Trainingseingabedaten und zugehörigen, d.h. damit verknüpften, Vergleichsausgabedaten basieren, wobei die trainierte Funktion zur Erzeugung von Trainingsausgabedaten auf die Trainingseingabedaten angewendet wird. Insbesondere können das Bestimmen und/oder das Anpassen auf einem Vergleich der Trainingsausgabedaten und der Trainingsvergleichsdaten basieren. Im Allgemeinen wird auch eine trainierbare Funktion, d.h. eine Funktion mit noch nicht angepassten einen oder mehreren Parametern, als trainierte Funktion bezeichnet.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk, wobei die Kantengewichte des künstlichen neuronalen Netzwerks den Parametern der trainierten Funktion entsprechen. Anstatt des Begriffs "neuronales Netzwerk" kann auch der Begriff "neuronales Netz" verwendet werden. Insbesondere kann eine trainierte Funktion auch ein tiefes künstliches neuronales Netzwerk sein (engl. deep neural network, deep artificial neural network). Im Rahmen dieses alternativen Verfahrens kann insbesondere ein neuronales Netz im Form eines sogenannten U-Net (siehe beispielsweise: Ronneberger O, Fischer P and Brox T. U-Net: Convolutional Networks for Biomedical Image Segmentation, http://arxiv.org/abs/1505.04597) eingesetzt werden. Es können jedoch auch andere aus dem Stand der Technik bekannte Architekturen genutzt werden, insbesondere solche, welche auf einem Deep Learning Model mit einer Encoder-Decoder Architektur basieren.

Die trainierte Funktion kann insbesondere mittels einer Rückpropagation trainiert sein. Zunächst können Trainingsausgabedaten durch Anwendung der trainierten Funktion auf Trainingseingabedaten bestimmt werden. Hiernach kann eine Abweichung zwischen den Trainingsausgabedaten und den Trainingsvergleichsdaten durch Anwendung einer Fehlerfunktion auf die Trainingsausgabedaten und die Trainingsvergleichsdaten ermittelt werden. Ferner kann zumindest ein Parameter, insbesondere eine Gewichtung, der trainierten Funktion, insbesondere des neuronalen Netzwerks, basierend auf einem Gradienten der Fehlerfunktion bezüglich des zumindest einen Parameters der trainierten Funktion iterativ angepasst werden. Hierdurch kann die Abweichung zwischen den Trainingsausgabedaten und den Trainingsvergleichsdaten während des Trainings der trainierten Funktion vorteilhafterweise minimiert werden.

Vorteilhafterweise weist die trainierte Funktion, insbesondere das neuronale Netzwerk, eine Eingabeschicht und eine Ausgabeschicht auf. Dabei kann die Eingabeschicht zum Empfang von Eingabedaten ausgebildet sein. Ferner kann die Ausgabeschicht zur Bereitstellung von Ausgabedaten ausgebildet sein. Dabei kann die Eingabeschicht und/oder die Ausgabeschicht jeweils mehrere Kanäle, insbesondere Neuronen, umfassen.

Die Eingabedaten für die trainierte Funktion können zumindest die zumindest drei CT-Bilddatensätze eines mCTA-Bilddatensatz des Patienten umfassen. Die Ausgabedaten können insbesondere den virtuellen, nicht-kontrastierte CT-Bilddatensatz umfassen.

Für ein Training der trainierten Funktion müssen Trainingsdaten bereitgestellt werden. Die Trainingsdaten umfassen den zuvor bereits erwähnten virtuellen, nicht-kontrastierten Trainings-Bilddatensatz als Trainingsausgabedaten und den nicht-kontrastierten Vergleichs-Bilddatensatz als Trainingsvergleichsdaten.

Für das Training werden die Trainingseingabedaten bereitgestellt, die trainierte Funktion auf die bereitgestellten Trainingseingabedaten angewendet, um Trainingsausgabedaten zu erhalten und schließend basierend auf einem Vergleich der Trainingsausgabedaten mit dazu zugehörigen Trainingsvergleichsdaten Parameter der trainierten Funktion angepasst. Die Trainingseingabedaten und die Trainingsvergleichsdaten sind miteinander verknüpft. Insbesondere können annotierte Trainingseingabedaten eingesetzt werden.

Trainingseingabedaten, mittels welchen durch Anwendung der trainierten Funktion auf die Trainingseingabedaten in der Trainingsphase Trainingsausgabedaten bestimmt werden, und Trainingsvergleichsdaten können auf verschiedene Arten erzeugt bzw. bereitgestellt werden.

In einer vorteilhaft einfachen Form können die Trainingseingabedaten am Patienten oder mit Phantomen gemessenen mCTA-Bilddatensätzen entsprechen, wobei Trainingsvergleichsdaten als zusätzlicher nicht-kontrastierter Bilddatensatz bereitgestellt werden können, welche bei den gleichen Aufnahmeparametern wie der gemessene mCTA-Bilddatensatz jedoch ohne Kontrastmitteleinfluss erfasst wurde. Dies stellt eine vorteilhaft einfache und direkte Trainingsvariante dar. Jedoch geht dies nachteilig mit zusätzlichen erforderlichen Messungen und damit Aufwand einher, insbesondere in Anbetracht der Tatsache, dass für das Training ein umfangreicher Satz an Trainingsdaten zur Verfügung gestellt sein sollte. Dies ist insbesondere bei Messungen an Patienten außerdem verbunden mit einer zusätzlichen Strahlungsbelastung.

In einer weiteren Variante können die Trainingsdaten zuerst basierend auf bereits vorhanden CTP-Daten nachgebildet werden. Nach einer ersten Trainingsphase mit solchen nachgebildeten Daten, könnte in einer zweiten Trainingsphase eine Feinjustierung auf tatsächlichen mCTA-Bilddatensätzen und dazu passenden, nicht-kontrastierten CT-Bilddatensätzen erfolgen. Dies ist vorteilhaft, da für eine Nachjustierung ein deutlich geringerer Umfang an Trainingsdaten bereitgestellt werden müsste und sich damit der Aufwand und eine zusätzliche Strahlenbelastung in einem geringeren Umfang im Vergleich zu einem Training direkt basierend auf mCTA-Bilddatensätzen bewegt.

Das heißt, in dieser Variante werden für das Training nachgebildete mCTA-Bilddatensätze basierend auf CTP-Bilddatensätzen als Trainingseingabedatensatz bereitgestellt und ebenso ein nachgebildeter nicht-kontrastierter Bilddatensatz basierend auf CTP-Bilddatensätzen als Trainingsvergleichsdatensatz.

Die nachgebildeten mCTA-Bilddatensätze basierend auf den CTP-Bilddatensätzen basieren auf der Überlegung, dass eine Teilmenge eines zeitaufgelösten CTP-Bilddatensatzes, welche ausgewählten Zeitpunkten zugeordnet ist, ähnliche Information wie die zumindest drei CT-Bilddatensätze eines mCTA-Bilddatensatzes abbildet. Das heißt, es werden gezielt zumindest drei Bilddatensätze eines CTP-Bilddatensatzes ausgewählt, welche im Wesentlichen den abgebildeten Phasen eines mCTA-Bilddatensatzes entsprechen, wohingegen als Trainingsvergleichsdatensatz eines der zeitlichen ersten Bilddatensätze, insbesondere der erste Bilddatensatz, eines CTP-Bilddatensatzes verwendet werden kann, in welchem in der Regel noch keine Einwirkung von Kontrastmittel im interessierenden Abbildungsbereich vorliegt. Basierend auf diesen Bilddaten kann in einer ersten Trainingsphase ein Vortraining der trainierten Funktion durchgeführt werden.

Anschließend kann in einer zweiten Trainingsphase eine Feinjustierung erfolgen. Hier erfolgt ein weiteres Training der vortrainierten Funktion basierend auf bereitgestellten, gemessenen mCTA-Bilddatensätzen als Trainingseingabedaten und zugehörigen nicht-kontrastierten CT-Bilddatensätzen als Trainingsvergleichsdaten. Vorteilhaft ist lediglich nur ein geringer Umfang an mCTA-Bilddaten und zugehörigen nicht-kontrastierten CT-Bilddatensätzen für eine Feinjustierung notwendig, wobei für ein Großteil des Trainings breit verfügbare, vorhandene CTP-Bilddatensätze verwendet werden können.

In einer weiteren Variante kann auch eine solche Feinjustierung ggf. umgangen werden, wenn weitere Schritte zur Unterstützung der Übertragbarkeit auf mCTA-Bilddatensätze unternommen werden. Ein solcher Schritt könnte darin bestehen, die trainierte Funktion so zu trainieren, dass es die Differenz zwischen der Eingabe und der gewünschten Ausgabe anstelle der Ausgabe selbst ausgibt (bekannt als Residual Learning). Dies kann vorteilhaft nicht nur unerwünschte Komplexität im Allgemeinen reduzieren, sondern insbesondere verhindert, dass die trainierte Funktion zusätzlich zur Entfernung des Kontrastmittelkontrasts auch das Erscheinungsbild von Perfusionsbilddatensätzen reproduziert. Das heißt in dieser Variante erfolgt ein Training ebenfalls basierend auf nachgebildete mCTA-Bilddatensätzen basierend auf CTP-Bilddatensätzen und nachgebildeten nicht-kontrastierter Bilddatensatz basierend auf CTP-Bilddatensätzen, wobei ein Verfahren des Residual Learnings eingesetzt wird. Hier sei beispielsweise auf Shen, Wei und Liu, Rujie "Learning Residual Images for Face Attribute Manipulation" (https://doi.org/10.48550/arxiv.1612.05363) verwiesen.

In allen hier beschriebenen Varianten für den Einsatz einer trainierten Funktion kann außerdem als zusätzlicher Eingangsdatensatz ein nativer, nicht-kontrastierter Bilddatensatz, wie er üblicherweise vorliegt, als eine Hilfsvariable genutzt werden. Dieser stellt, trotz unterschiedlicher Aufnahmeparameter im Vergleich zu den mCTA-Bilddatensätzen, zusätzliche komplementäre Patienteninformation bereit, beispielsweise ein besserer Weichteilkontrast. Diese Information kann als zusätzlicher Eingabedatensatz in die trainierte Funktion zu einer akkurateren Abbildung durch die trainierte Funktion führen. Ist eine solche Eingabe vorgesehen, muss dies entsprechend auch bereits beim Training der trainierten Funktion berücksichtigt werden.

Im Rahmen der Erfindung können außerdem Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das Computertomographie-Gerät das Computertomographie-Gerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von beispielhaften Ausführungsformen unter Hinweis auf die beigefügten Figuren erläutert. Die Darstellung in den Figuren ist schematisch, stark vereinfacht und nicht zwingend maßstabsgetreu. Es zeigen:
Fig. 1 ein Verfahren zur Bereitstellung eines virtuellen, nicht-kontrastierten Bilddatensatzes eines Patienten in einer ersten Ausführungsform,
Fig. 2 ein Verfahren zur Bereitstellung eines virtuellen, nicht-kontrastierten Bilddatensatzes eines Patienten in einer zweiten Ausführungsform,
Fig. 3 ein Verfahren zur Bereitstellung eines virtuellen, nicht-kontrastierten Bilddatensatzes eines Patienten in einer dritten Ausführungsform,
Fig. 4 eine Vorrichtung zur Bereitstellung eines virtuellen, nicht-kontrastierten Bilddatensatzes eines Patienten, und
Fig. 5 ein Computertomographie-Gerät aufweisend eine Vorrichtung zur Bereitstellung eines virtuellen, nicht-kontrastierten Bilddatensatzes eines Patienten.

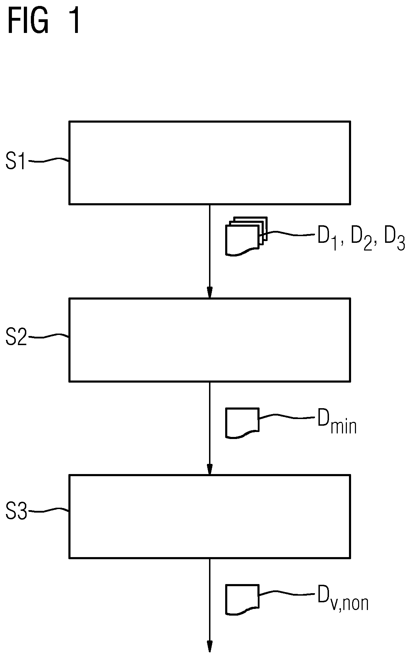

Fig. 1 zeigt einen schematischen Ablauf eines Verfahrens zur Bereitstellung eines virtuellen, nicht-kontrastierten Bilddatensatzes D_{v,non} eines Patienten 39 in einer ersten Ausführungsform.

Das Verfahren umfasst den Schritt des Bereitstellens S1 eines Mehrphasen CT-Angiographie-Bilddatensatzes des Patienten 39 umfassend zumindest drei CT-Bilddatensätze D₁, D₂, D₃, welche einen Abbildungsbereich des Patienten zu drei verschiedenen Zeitpunkten relativ zu einer Kontrastmittelgabe abbilden. Das Verfahren umfasst weiterhin den Schritt des Bildens S2 eines Minimumintensitätsbilddatensatzes Dₘᵢₙ des Abbildungsbereichs basierend auf den zumindest drei CT-Bilddatensätzen D₁, D₂, D₃, wobei der Bildwert eines Bildpunkts des Minimumintensitätsbilddatensatzes Dₘᵢₙ jeweils auf dem minimalen Wert unter den Bildwerten der im Abbildungsbereich örtlich korrespondierenden Bildpunkte in den zumindest drei CT-Bilddatensätzen D₁, D₂, D₃ basiert.

Das Verfahren umfasst weiterhin den Schritt des Ausgebens S3 des virtuellen, nicht-kontrastierten Bilddatensatzes D_{v,non} basierend auf dem Minimumintensitätsbilddatensatz Dₘᵢₙ.

Der Abbildungsbereich des Patienten 39, welcher von den zumindest drei CT-Bilddatensätzen D₁, D₂, D₃, ebenso wie von dem virtuellen, nicht-kontrastierten CT-Bilddatensatz D_{v,non} und dem Minimumintensitätsbilddatensatz Dₘᵢₙ zumindest abgebildet wird, kann einen anatomischen und/oder räumlichen Bereich des Patienten 39 umfassen, der einen vorbestimmten Gewebebereich und/oder einen für eine Diagnose notwendigen räumlichen Bereich umfasst. So umfasst der Abbildungsbereich bei einem Schlaganfallpatienten insbesondere das Gehirn des Patienten 39. Bei anderer Anwendung kann der Abbildungsbereich auch anders sein.

Die Aufnahmebereiche der jeweiligen Bilddatensätze können sich unterscheiden. Bei der Anwendung des Verfahrens im Bereich der Schlaganfalldiagnostik unterscheidet sich beispielsweise der Aufnahmebereich des ersten CT-Bilddatensatzes D₁ der zumindest drei CT-Bilddatensätze D₁, D₂, D₃ von einem Aufnahmebereich des zweiten CT-Bilddatensatzes D₂ und/oder dritten CT-Bilddatensatzes D₃ der zumindest drei CT-Bilddatensätze D₁, D₂, D₃ insbesondere in der Art, dass der Aufnahmebereich des ersten CT-Bilddatensatzes D₁ zumindest den Patienten 39 vom Aortenbogen bis zum Scheitel umfassen, wobei der Aufnahmebereich des zweiten CT-Bilddatensatzes D₂ und/oder der Aufnahmebereich des dritten CT-Bilddatensatzes D₃ zumindest den Patienten 39 von der Schädelbasis bis zum Scheitel abbildet.

Bei unterschiedlichen Aufnahmebereichen kann das Verfahren insbesondere umfassen, dass beim Bilden des Minimumintensitätsbilddatensatzes Dₘᵢₙ eine Einschränkung der CT-Bilddatensätze auf den gewünschten Abbildungsbereich des virtuellen, nicht-kontrastierten Bilddatensatzes D_{v,non} erfolgt.

Werden wie oben beschrieben der mCTA-Bilddatensatz derart bereitgestellt, dass der Aufnahmebereich des ersten CT-Bilddatensatzes D₁ zumindest den Patienten 39 vom Aortenbogen bis zum Scheitel umfassen, wobei der Aufnahmebereich des zweiten CT-Bilddatensatzes D₂ und der Aufnahmebereich des dritten CT-Bilddatensatzes D₃ zumindest den Patienten 39 von der Schädelbasis bis zum Scheitel abbildet, so kann für das Bilden des Minimumintensitätsbilddatensatz Dₘᵢₙ ein vorläufiger Bilddatensatz D_{base} erzeugt werden, welcher lediglich die Größe des zweiten und dritten CT-Bilddatensatz aufweist. Der Minimumintensitätsbilddatensatz kann dann erzeugt werden, indem der vorläufige Bilddatensatz D_{base} mit jeweiligen durch eine Minimumsintensitätsprojektion basierend auf dem mCTA-Bilddatensatz ermittelten Bildwerten befüllt wird und als Minimumintensitätsbilddatensatz Dₘᵢₙ bereitgestellt wird. Das bedeutet die Bildwerte V_{base} können im Wesentlichen einem min (V₁, V₂,V₃) entsprechen, wobei die Vₓ Bildpunktwerten an korrespondierenden Positionen in Dₓ mit x e{base,1,2,3} entsprechen. Örtlich korrespondieren bedeutet dabei, dass örtlich korrespondierende Bildpunkte in den zumindest drei CT-Bilddatensätzen D₁, D₂, D₃ und entsprechen auch im Minimumintensitätsbilddatensatz Dₘᵢₙ jeweils den gleichen Bildbereich des Patienten 39 abbilden. Dieses Vorgehen gilt in gleicher Weise für andere Aufnahme- und Abbildungsbereiche.

Die zumindest drei CT-Bilddatensätze D₁, D₂, D₃ bilden jeweils den Zustand der Kontrastmittelverteilung im Patienten zu verschiedenen Zeitpunkten relativ zu der Kontrastmittelgabe ab. In einer vorteilhaften Verfahrensvariante beträgt dabei der relative Zeitabstand zwischen dem zeitlich ersten CT-Bilddatensatzes D₁ und dem zweiten CT-Bilddatensatzes D₂ der zumindest drei CT-Bilddatensätze D₁, D₂, D₃ bzw. der relative Zeitabstand zwischen dem zeitlich zweiten CT-Bilddatensatzes D₂ und dem dritten CT-Bilddatensatzes D₃ der zumindest drei CT-Bilddatensätze D₁, D₂, D₃ mindestens 5s beträgt. Für die mCTA im Rahmen einer Diagnostik im Rahmen eines Schlaganfalls ist ein Zeitabstand von 7-10s besonders vorteilhaft. Insbesondere wird die zeitliche Verzögerung zwischen den jeweiligen Starts der Datenaufnahme derart gewählt, dass die CT-Bilddatensätze D₁, D₂, D₃ für eine nachfolgende Diagnostik geeignet sind. Im Gegensatz dazu beträgt eine Zeitdifferenz zwischen Bilddatensätzen einer CTP lediglich 1 bis 2 s oder weniger.

Da die zumindest drei CT-Bilddatensätze D₁, D₂, D₃ den Abbildungsbereich jeweils zu unterschiedlichen Zeitpunkten relativ zu der Kontrastmittelgabe darstellen, ist die Annahme, dass für jeden abgebildeten Bereich bzw. Gewebetyp im Abbildungsbereich eine Abbildung in einer der drei CT-Bilddatensätze D₁, D₂, D₃ vorhanden ist, in welcher dieser bzw. dieses nicht von Kontrastmittel beeinflusst ist, so dass der Bildbereich desjenigen CT-Bilddatensatzes, für welchen das zutrifft für diesen Bereich als nicht-kontrastiertes Bild genutzt werden kann. Es kann dabei dann weiter ausgegangen werden, dass der jeweilige minimale Bildwert für einen korrespondierenden Bildpunkt bzw. Voxel in den zumindest drei CT-Bilddatensätze D₁, D₂, D₃, demjenigen entspricht, der nicht von Kontrastmittel beeinflusst ist, da das Kontrastmittel zu einer Erhöhung des Bildwertes relativ zu einem Ausgangswert führen würde.

Vorteilhaft wird damit ein virtueller, nicht-kontrastierter CT-Bilddatensatz bereitgestellt, welche zum bereitgestellten mCTA-Bilddatensatz passend ist.

Basierend auf den bereitgestellten Mehrphasen CT-Angiographie-Bilddatensatzes des Patienten und dem zughörigen nun bereitgestellten virtuellen, nicht-kontrastierten Bilddatensatz D_{v,non} kann dann in weiteren Schritten ein verbesserter Perfusionsbilddatensatz erzeugt werden, insbesondere sogenannte "Perfusionskarten", z. B. CBF oder CBV.

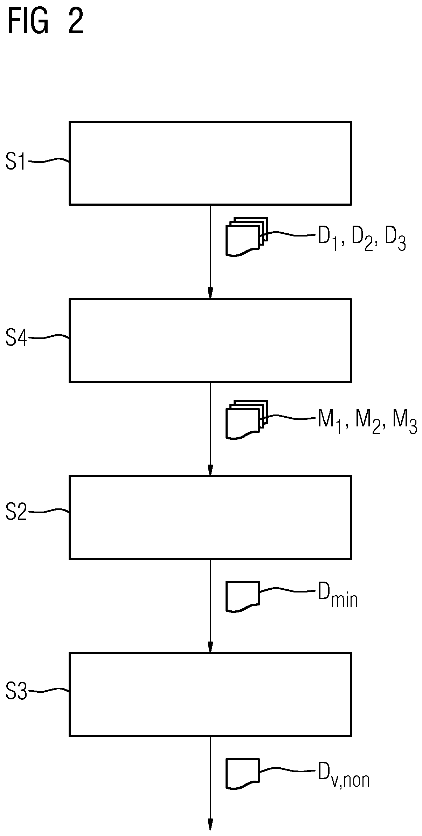

Fig. 2 zeigt ein Verfahren zur Bereitstellung eines virtuellen, nicht-kontrastierten Bilddatensatzes D_{v,non} eines Patienten 39 in einer zweiten Ausführungsform. Die S1, S2 und S3 können im Wesentlichen den im Rahmen der Beschreibung zu Fig. 1 erläuterten Schritten S1, S2 und S3 entsprechen.

Diese Verfahrensvariante umfasst jedoch weiterhin den Schritt des Durchführens S4 einer Bewegungskorrektur der zumindest drei CT-Bilddatensätze D₁, D₂, D₃, wodurch bewegungskorrigierte CT-Bilddatensätze M₁, M₂, M₃ bereitgestellt werden. Basierend auf diesen wird im anschließenden Schritt S2 dann der Minimumintensitätsbilddatensatz Dₘᵢₙ gebildet.

Für eine Bewegungskorrektur wie sie im Rahmen des Verfahrens eingesetzt werden kann gibt es verschiedene Möglichkeiten, welche dem Fachmann aus dem Stand der Technik bekannt sind. Auf Beispiele wurde bereits in der allgemeinen Beschreibung hingewiesen. Insbesondere kann eine bekannte rigide Registrierung der zumindest drei CT-Bilddatensätze D₁, D₂, D₃ genutzt werden.

Vorteilhaft kann eine Bewegungskorrektur Bewegungen des Patienten 39 zwischen der Messdatenaufnahme für die zumindest drei CT-Bilddatensätze D₁, D₂, D₃ Rechnung tragen und deren Auswirkung verringern und so ein verbesserter Minimumintensitätsbilddatensatz Dₘᵢₙ bereitgestellt werden.

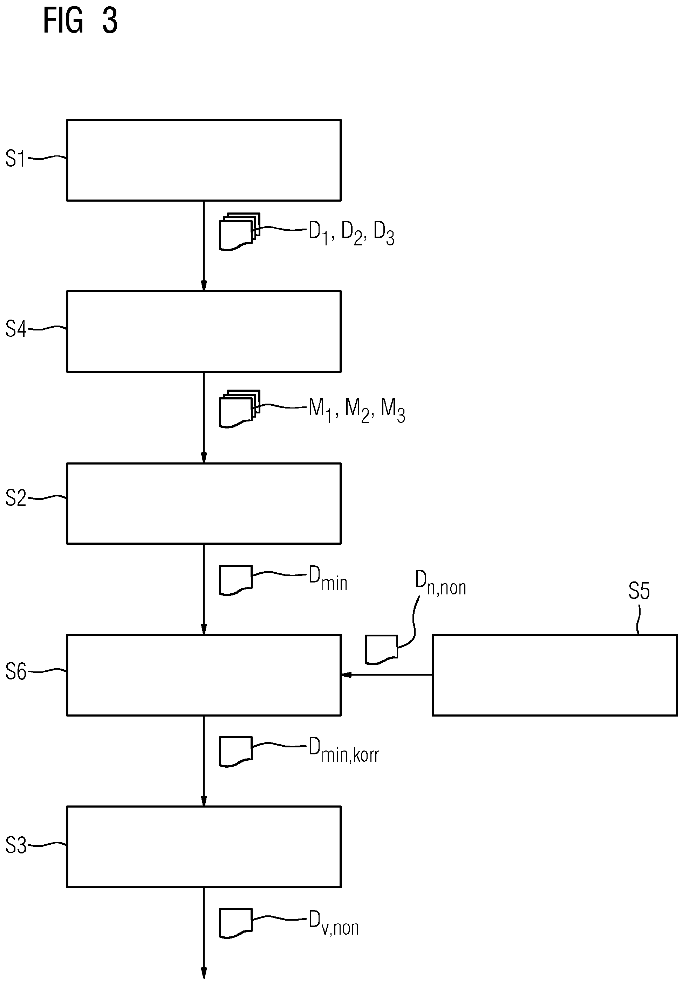

Fig. 3 zeig eine weitere Variante eines Verfahrens zur Bereitstellung eines virtuellen, nicht-kontrastierten Bilddatensatzes D_{v,non} eines Patienten 39. Zusätzlich zu der in Fig. 2 beschriebenen Variante umfasst diese Ausbildung außerdem den Schritt des Bereitstellens S5 eines nativen, nicht-kontrastierten CT-Bilddatensatzes D_{n,non}, welcher einen Abbildungsbereich des Patienten 39 vor Kontrastmittelgabe abbildet und welcher bei Aufnahmeparametern aufgenommen wurde, welche sich von den Aufnahmeparametern des mCTA-Bilddatensatz unterscheiden. In einem weiteren Schritt des Korrigieren S6 wird der Minimumintensitätsbilddatensatz Dₘᵢₙ unter Einsatz des nativen, nicht-kontrastierten CT-Bilddatensatzes D_{n,non} korrigiert, wodurch ein korrigierter Minimumintensitätsbilddatensatz D_{min, korr} bereitgestellt wird.

Der im Schritt des Ausgebens S5 ausgebende, virtuelle, nicht-kontrastierte Bilddatensatz D_{v,non} basiert dann auf dem korrigierten Minimumintensitätsbilddatensatz D_{min, korr}.

Auch in diesem Fall kann vor einem Vergleich eine Bewegungskorrektur durchgeführt werden, um mögliche Bewegungen des Patienten zwischen der Akquisition des nativen, nicht-kontrastierten CT-Bilddatensatzes und der dem Minimumintensitätsbilddatensatz Dₘᵢₙ zu Grunde liegenden zumindest drei CT-Bilddatensätze D₁, D₂, D₃ Rechnung zu tragen.

Dabei kann der Schritt des Korrigieren S6 umfassen, Bildbereiche im Minimumintensitätsbilddatensatz Dₘᵢₙ zu bestimmen, welche einen bestimmten Rauschwert überschreiten oder welche einen bestimmten Intensitätswert unterschreiten, und basierend auf einem Vergleich der bestimmten Bildbereiche mit örtlich korrespondierenden Bildbereichen in dem nativen, nicht-kontrastierten CT-Bilddatensatz D_{n,non} zumindest einen Korrekturwert zu bestimmen. Der zumindest eine Korrekturwert wird dann auf den nicht korrigierten Minimumintensitätsbilddatensatz Dₘᵢₙ angewendet.

Ebenso ist es möglich, dass für die Korrektur statt im Minimumintensitätsbilddatensatz Dₘᵢₙ Bildbereiche in zumindest einem der zumindest drei CT-Bilddatensätze D₁, D₂, D₃, bestimmt werden, welche einen bestimmten Rauschwert überschreiten oder welche einen bestimmten Intensitätswert unterschreiten, und basierend auf einem Vergleich zwischen jeweils dazu örtlich korrespondierenden Bildbereichen im Minimumintensitätsbilddatensatz Dₘᵢₙ und im nativen, nicht-kontrastierten CT-Bilddatensatz D_{n,non} zumindest ein Korrekturwert für die bestimmten Bildbereiche im Minimumintensitätsbilddatensatz Dₘᵢₙ bestimmt wird.

Alternativ kann der Schritt des Korrigierens S6 auch umfassen, basierend auf den zumindest drei CT-Bilddatensätzen D₁, D₂, D₃ des mCTA-Bilddatensatzes des Patienten 39 einen Perfusionsbilddatensatz zu erstellen, Bildbereiche des Perfusionsbilddatensatzes mit lokal erhöhter Kontrastmittelaufnahme zu identifizieren, und basierend auf einem Vergleich zwischen dazu örtlich korrespondierender Bildbereiche im Minimumintensitätsbilddatensatz Dₘᵢₙ und örtlich korrespondierender Bildbereichen in dem nativen, nicht-kontrastierten CT-Bilddatensatz D_{n,non} zumindest einen Korrekturwert zu bestimmen. Der zumindest eine Korrekturwert wird dann auf den nicht korrigierten Minimumintensitätsbilddatensatz angewendet.

Unabhängig von der Ausführung, d.h. wie die Bildbereiche bestimmt werden, kann dann der Korrekturwert insbesondere auf einem Verhältnis der Bildwerte in den bestimmten Bildbereichen des Minimumintensitätsbilddatensatz Dₘᵢₙ bzw. des nativen, nicht-kontrastierten CT-Bilddatensatzes D_{n,non} zu Bildwerten in einer Umgebung der bestimmten Bildbereiche des Minimumintensitätsbilddatensatz Dₘᵢₙ bzw. des nativen, nicht-kontrastierten CT-Bilddatensatzes D_{n,non} basieren. Der Vergleich kann insbesondere umfassen, einen Kontrastwert zwischen vorliegenden Strukturen oder Bildbereichen zu umliegenden Strukturen oder Bereichen zu vergleichen. Ist ein Verhältnis in beiden Bilddatensätzen ähnlich oder innerhalb eines Erwartungsbereichs, so ist die Wahrscheinlichkeit größer, dass hier kein Artefakt, sondern die Bildwerte tatsächlich so vorliegen. Unterscheidet sich das Verhältnis signifikant oder liegt außerhalb der Erwartungen, so werden die Bildwerte in den zuvor bestimmten Bildbereichen des Minimumintensitätsbilddatensatzes Dₘᵢₙ mittels zumindest eines Korrekturwerts angepasst, beispielsweise in einer Art, so dass daraus ein Verhältnis ähnlich oder gleich wie im nativen, nicht-kontrastierten CT-Bilddatensatz resultiert oder innerhalb des Erwartungsbereichs liegt.

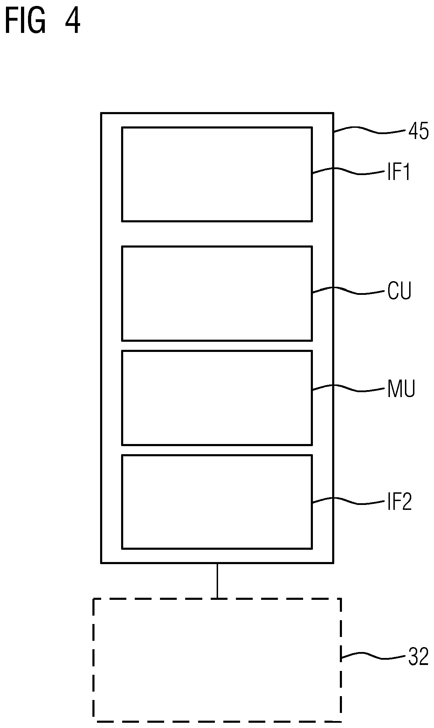

Fig. 4 zeigt eine Vorrichtung 45 zur Bereitstellung eines virtuellen, nicht-kontrastierten Bilddatensatzes D_{v,non} eines Patienten umfassend
- eine erste Schnittstelle IF1, ausgebildet zumindest einen Mehrphasen CT-Bilddatensatzes des Patienten bereitzustellen, umfassend zumindest drei CT-Bilddatensätze D₁, D₂, D₃, welche jeweils einen Abbildungsbereich des Patienten zu drei verschiedenen Zeitpunkten relativ zu einer Kontrastmittelgabe abbilden
- eine Recheneinheit CU, ausgebildet einen Minimumintensitätsbilddatensatz Dₘᵢₙ des Abbildungsbereichs basierend auf den zumindest drei CT-Bilddatensätzen D₁, D₂, D₃ zu bilden, wobei der Bildwert eines Bildpunkts des Minimumintensitätsbilddatensatzes Dₘᵢₙ jeweils auf dem minimalen Wert unter den Bildwerten der im Abbildungsbereich örtlich korrespondierenden Bildpunkte in den zumindest drei CT-Bilddatensätzen D₁, D₂, D₃ basiert, und
- eine zweite Schnittstelle IF2, ausgebildet den virtuellen nicht-kontrastierten Bilddatensatz D_{v,non} basierend auf dem Minimumintensitätsbilddatensatz Dₘᵢₙ auszugeben.

Bei der Vorrichtung 45 bzw. der Recheneinheit CU kann es sich insbesondere um einen Computer, einen Mikrocontroller oder um einen integrierten Schaltkreis handeln. Alternativ kann es sich dabei um einen realen oder virtuellen Verbund von Computern handeln (ein englischer Fachbegriff für einen realen Verbund ist "Cluster", ein englischer Fachbegriff für einen virtuellen Verbund ist "Cloud"). Die Vorrichtung 45 kann auch als virtuelles System ausgebildet sein, das auf einem realen Computer oder einem realen oder virtuellen Verbund von Computern ausgeführt wird (engl. virtualization).

Bei einer Schnittstelle IF1, IF2 kann es sich um eine Hardware- oder Softwareschnittstelle handeln (beispielsweise PCI-Bus, USB oder Firewire). Eine Recheneinheit CU kann Hardware-Elemente oder Software-Elemente aufweisen, beispielsweise einen Mikroprozessor oder ein sogenanntes FPGA (englisches Akronym für "Field Programmable Gate Array").

Die Schnittstellen IF1, IF2 können insbesondere mehrere Unterschnittstellen umfassen. Mit anderen Worten können die Schnittstellen IF1, IF2 auch eine Vielzahl von Schnittstellen umfassen. Die Recheneinheit CU können insbesondere mehrere Unterrecheneinheiten umfassen, die unterschiedliche Schritte der jeweiligen Verfahren ausführen. Mit anderen Worten kann die Recheneinheit CU auch als Vielzahl von Recheneinheiten aufgefasst werden.

Die Vorrichtung 45 kann außerdem auch eine Speichereinheit MU umfassen. Eine Speichereinheit kann als nicht dauerhafte Arbeitsspeicher (Random Access Memory, kurz RAM) oder als dauerhafter Massenspeicher (Festplatte, USB-Stick, SD-Karte, Solid State Disk) realisiert sein.

Eine solche Vorrichtung 45 zur Bereitstellung eines virtuellen, nicht-kontrastierten Bilddatensatzes D_{v,non} eines Patienten 39 kann insbesondere dazu ausgebildet sein, die zuvor beschriebenen erfindungsgemäßen Verfahren zur Bereitstellung eines virtuellen, nicht-kontrastierten Bilddatensatzes D_{v,non} eines Patienten 39 und ihre Aspekte auszuführen. Die Vorrichtung 45 kann dazu ausgebildet sein, die Verfahren und ihre Aspekte auszuführen, indem die Schnittstellen IF1, IF2 und die Recheneinheit CU ausgebildet sind, die entsprechenden Verfahrensschritte auszuführen.

Die Vorrichtung kann weiterhin mit einem CT-Gerät 32 verbunden sein, welches ausgebildet ist Messdaten für einen mCTA-Bilddatensatz zu akquirieren.

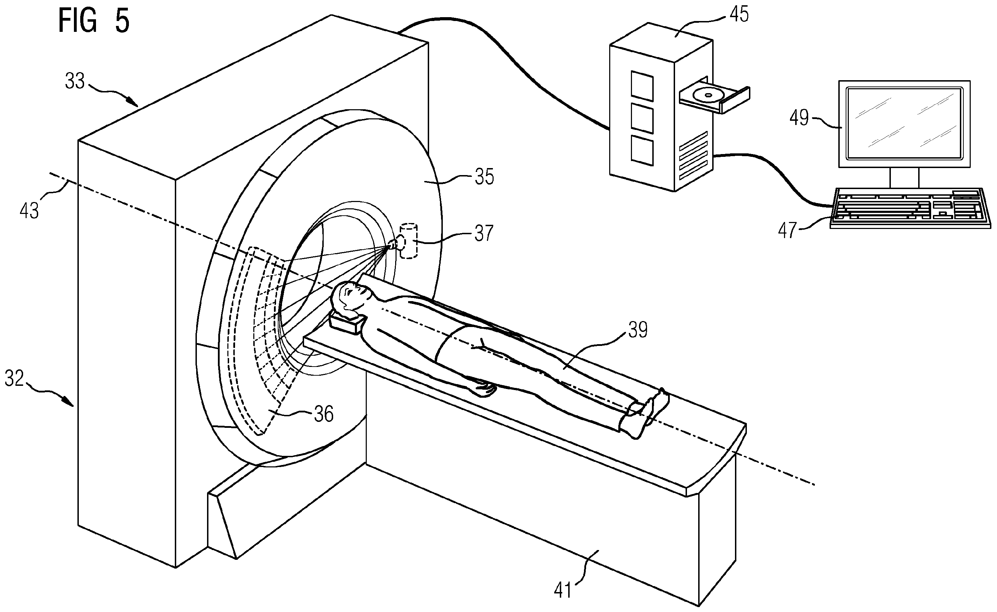

Fig. 5 zeigt ein erfindungsgemäßes CT Gerät 32. Das CT-Gerät weist eine Gantry 33 mit einem Rotor 35 auf. Der Rotor 35 umfasst zumindest eine Röntgenquelle 37, insbesondere eine Röntgenröhre, und in Gegenüberstellung dazu zumindest einen Röntgendetektor 2. Der Röntgendetektor 2 und die Strahlungsquelle 37 sind um eine gemeinsame Achse 43 (auch Rotationsachse genannt) rotierbar. Der Patient 39 ist auf einer Patientenliege 41 gelagert und ist entlang der Rotationsachse 43 durch die Gantry 33 bewegbar. Im Allgemeinen kann der Patient 39 beispielsweise einen tierischen Patienten und/oder einen menschlichen Patienten umfassen.

Üblicherweise werden aus einer Vielzahl an Projektionswinkeln während einer relativen Rotationsbewegung zwischen der Röntgenquelle 37 und dem Patienten 39 Messdaten in Form von einer Mehrzahl an (Roh-) Projektionsdatensätze des Patienten 39 aufgenommen, während der Patient 39 kontinuierlich oder sequentiell durch die Gantry 33 mittels der Patientenliege 41 bewegt wird. Anschließend kann basierend auf den Projektionsdatensätzen mittels eines mathematischen Verfahrens, beispielsweise umfassend eine gefilterte Rückprojektion oder ein iteratives Rekonstruktionsverfahren, ein Bilddatensatz des Abbildungsbereichs rekonstruiert werden.

Das CT-Gerät 32 umfasst ein Computersystem 45. Das Computersystem 45 kann außerdem eine Rekonstruktionseinheit zur Rekonstruktion von Bilddatensätzen basierend auf den von dem Bildgebungsgerät 32 ermittelten Messdaten umfassen. Das Computersystem 45 kann außerdem eine Steuereinheit zur Ansteuerung des CT-Geräts aufweisen. Das Computersystem 45 umfasst insbesondere eine Vorrichtung 45 zur Bereitstellung eines virtuellen, nicht-kontrastierten CT-Bilddatensatzes.

Die von dem Computersystem 45 umfasste Vorrichtung 45 zur Bereitstellung eines virtuellen, nicht-kontrastierten Bilddatensatzes D_{v,non} eines Patienten ist insbesondere ausgebildet ein erfindungsgemäßes Verfahren zur Erzeugung eines Ergebnisbilddatensatzes wie zuvor beschrieben durchzuführen.

Des Weiteren ist eine Eingabeeinrichtung 47 und eine Ausgabeeinrichtung 49 mit dem Computersystem 45 verbunden. Die Eingabeeinrichtung 47 und die Ausgabeeinrichtung 49 können beispielsweise eine Interaktion, beispielsweise eine manuelle Konfiguration, eine Bestätigung oder ein Auslösen eines Verfahrensschritts durch einen Anwender ermöglichen. Beispielsweise können dem Nutzer auf dem Ausgabevorrichtung 49 umfassend ein Monitor die zumindest drei CT-Bilddatensätze, der Minimumintensitätsbilddatensatz oder der virtuelle, nicht-kontrastierte CT-Bilddatensatz angezeigt werden.

## Patentansprüche

1. Verfahren zur Bereitstellung eines virtuellen, nicht-kontrastierten Bilddatensatzes (D_{v,non}) eines Patienten (39) umfassend die Schritte
- Bereitstellen (S1) eines Mehrphasen CT-Angiographie-Bilddatensatzes des Patienten (39) umfassend zumindest drei CT-Bilddatensätze (D₁, D₂, D₃), welche einen Abbildungsbereich des Patienten zu drei verschiedenen Zeitpunkten relativ zu einer Kontrastmittelgabe abbilden,
**gekennzeichnet durch**:
- Bilden (S2) eines Minimumintensitätsbilddatensatzes (Dₘᵢₙ) des Abbildungsbereichs basierend auf den zumindest drei CT-Bilddatensätzen (D₁, D₂, D₃), wobei der Bildwert eines Bildpunkts des Minimumintensitätsbilddatensatzes (Dₘᵢₙ) jeweils auf dem minimalen Wert unter den Bildwerten der im Abbildungsbereich örtlich korrespondierenden Bildpunkte in den zumindest drei CT-Bilddatensätzen (D₁, D₂, D₃) basiert, wobei der minimale Wert demjenigen Wert entspricht, welcher der höchsten Transmission an Röntgenstrahlung und damit der geringsten Absorption entspricht,
- Ausgeben (S3) des virtuellen, nicht-kontrastierten Bilddatensatzes (D_{v,non}) basierend auf dem Minimumintensitätsbilddatensatz (Dₘᵢₙ), wobei der virtuelle, nicht-kontrastierte CT-Bilddatensatz (D_{v,non}) mittels einer Recheneinheit basierend auf Messdaten erzeugt wird, welche mit Kontrastmittelgabe erfasst wurden.

2. Verfahren nach Anspruch 1, wobei der relative Zeitabstand zwischen dem zeitlich ersten CT-Bilddatensatzes (D₁) und dem zweiten CT-Bilddatensatzes (D₂) der zumindest drei CT-Bilddatensätze (D₁, D₂, D₃) bzw. der relative Zeitabstand zwischen dem zeitlich zweiten CT-Bilddatensatzes (D₂) und dem dritten CT-Bilddatensatzes (D₃) der zumindest drei CT-Bilddatensätze (D₁, D₂, D₃) mindestens 5s beträgt.

3. Verfahren nach einem der vorangehenden Ansprüche, wobei der Abbildungsbereich das Gehirn des Patienten (39) umfasst.

4. Verfahren nach einem der vorangehenden Ansprüche, wobei sich ein Aufnahmebereich des ersten CT-Bilddatensatzes (D₁) der zumindest drei CT-Bilddatensätze (D₁, D₂, D₃) von einem Aufnahmebereich des zweiten CT-Bilddatensatzes (D₂) und/oder dritten CT-Bilddatensatzes (D₃) der zumindest drei CT-Bilddatensätze (D₁, D₂, D₃) unterscheidet.

5. Verfahren nach Anspruch 4, wobei der Aufnahmebereich des ersten CT-Bilddatensatzes (D₁) zumindest den Patienten (39) vom Aortenbogen bis zum Scheitel umfasst, und wobei der Aufnahmebereich des zweiten CT-Bilddatensatzes (D₂) und/oder der Aufnahmebereich des dritten CT-Bilddatensatzes (D₃) zumindest den Patienten (39) von der Schädelbasis bis zum Scheitel abbildet.

6. Verfahren nach einem der vorangehenden Ansprüche, außerdem umfassend den Schritt:
- Durchführen (S4) einer Bewegungskorrektur der zumindest drei CT-Bilddatensätze (D₁, D₂, D₃), wodurch bewegungskorrigierte CT-Bilddatensätze (M₁, M₂, M₃) bereitgestellt werden, und
wobei der Minimumintensitätsbilddatensatz (Dₘᵢₙ) basierend auf den bewegungskorrigierten CT-Bilddatensätzen (M₁, M₂, M₃) gebildet wird.

7. Verfahren nach einem der vorangehenden Ansprüche, außerdem umfassend die Schritte
- Bereitstellen (S5) eines nativen, nicht-kontrastierten CT-Bilddatensatzes (D_{n,non}), welcher einen Abbildungsbereich des Patienten vor Kontrastmittelgabe abbildet, und
- Korrigieren (S6) des Minimumintensitätsbilddatensatz (Dₘᵢₙ) unter Einsatz des nativen, nicht-kontrastierten CT-Bilddatensatzes (D_{n,non}), wodurch ein korrigierter Minimumintensitätsbilddatensatz (D_{min,korr}) bereitgestellt wird, und wobei
der ausgegebene virtuelle, nicht-kontrastierte Bilddatensatz (D_{v,non}) auf dem korrigierten Minimumintensitätsbilddatensatz (D_{min,korr}) basiert.

8. Verfahren nach Anspruch 7, wobei der Schritt des Korrigieren (S6) umfasst, Bildbereiche im Minimumintensitätsbilddatensatz (Dₘᵢₙ) oder zumindest einem der zumindest drei CT-Bilddatensätze zu bestimmen, welche einen bestimmten Rauschwert überschreiten oder welche einen bestimmten Intensitätswert unterschreiten, und basierend auf einem Vergleich zwischen jeweils dazu örtlich korrespondierenden Bildbereichen im Minimumintensitätsbilddatensatz (Dₘᵢₙ) und im nativen, nicht-kontrastierten CT-Bilddatensatz (D_{n,non}) einen Korrekturwert zu bestimmen.

9. Verfahren nach Anspruch 7, wobei der Schritt des Korrigierens (S6) umfasst, basierend auf den zumindest drei CT-Bilddatensätzen (D₁, D₂, D₃) des Mehrphasen CT-Angiographie-Bilddatensatzes des Patienten (39) einen Perfusionsbilddatensatz zu erstellen, Bildbereiche des Perfusionsbilddatensatzes mit lokal erhöhter Kontrastmittelaufnahme zu identifizieren, und basierend auf einem Vergleich zwischen dazu örtlich korrespondierender Bildbereiche im Minimumintensitätsbilddatensatz (Dₘᵢₙ) und örtlich korrespondierender Bildbereichen in dem nativen, nicht-kontrastierten CT-Bilddatensatz (D_{n,non}) einen Korrekturwert zu bestimmen.

10. Verfahren nach einem der Ansprüche 8 oder 9, wobei der Korrekturwert auf einem Verhältnis der Bildwerte in den bestimmten Bildbereichen des Minimumintensitätsbilddatensatz (Dₘᵢₙ) bzw. des nativen, nicht-kontrastierten CT-Bilddatensatzes (D_{n,non}) zu Bildwerten in einer Umgebung der bestimmten Bildbereiche des Minimumintensitätsbilddatensatz (Dₘᵢₙ) bzw. des nativen, nicht-kontrastierten CT-Bilddatensatzes (D_{n,non}) basiert.

11. Verfahren zur Bereitstellung eines Perfusionsbilddatensatzes, wobei
- ein Mehrphasen CT-Angiographie-Bilddatensatz des Patienten (39) umfassend zumindest drei CT-Bilddatensätze (D₁, D₂, D₃), welche einen Abbildungsbereich des Patienten zu drei verschiedenen Zeitpunkten relativ zu einer Kontrastmittelgabe abbilden, bereitgestellt wird,
- nach einem der vorangehenden Ansprüchen basierend auf den zumindest drei CT-Bilddatensätzen (D₁, D₂, D₃) ein virtueller, nicht-kontrastierter CT-Bilddatensatz (D_{v,non}) des Abbildungsbereichs bereitgestellt wird, und
- basierend auf den zumindest drei CT-Bilddatensätze (D₁, D₂, D₃) und dem virtuellen, nicht-kontrastierten CT-Bilddatensatz (D_{v,non}) ein Perfusionsbilddatensatz erzeugt und bereitgestellt wird.

12. Vorrichtung (45) zur Bereitstellung eines virtuellen, nicht-kontrastierten Bilddatensatzes (D_{v,non}) eines Patienten umfassend
- eine erste Schnittstelle (IF1), ausgebildet zumindest einen Mehrphasen CT-Bilddatensatzes des Patienten bereitzustellen, umfassend zumindest drei CT-Bilddatensätze (D₁, D₂, D₃), welche jeweils einen Abbildungsbereich des Patienten zu drei verschiedenen Zeitpunkten relativ zu einer Kontrastmittelgabe abbilden
- eine Recheneinheit (CU), ausgebildet einen Minimumintensitätsbilddatensatz (Dₘᵢₙ) des Abbildungsbereichs basierend auf den zumindest drei CT-Bilddatensätzen (D₁, D₂, D₃) zu bilden, wobei der Bildwert eines Bildpunkts des Minimumintensitätsbilddatensatzes (Dₘᵢₙ) jeweils auf dem minimalen Wert unter den Bildwerten der im Abbildungsbereich örtlich korrespondierenden Bildpunkte in den zumindest drei CT-Bilddatensätzen (D₁, D₂, D₃) basiert, wobei der minimale Wert demjenigen Wert entspricht, welcher der höchsten Transmission an Röntgenstrahlung und damit der geringsten Absorption entspricht, und
- eine zweite Schnittstelle (IF2), ausgebildet den virtuellen nicht-kontrastierten Bilddatensatz (D_{v,non}) basierend auf dem Minimumintensitätsbilddatensatz (Dₘᵢₙ) auszugeben, wobei der virtuelle, nicht-kontrastierte CT-Bilddatensatz (D_{v,non}) mittels einer Recheneinheit basierend auf Messdaten erzeugt wird, welche mit Kontrastmittelgabe erfasst wurden.

13. Computertomographie-Gerät (32) aufweisend eine Vorrichtung (45) gemäß Anspruch 12.

14. Computerprogrammprodukt mit einem Computerprogramm, welches direkt in einen Speicher (MU) einer Vorrichtung (45) zur Bereitstellung eines virtuellen, nicht-kontrastierten Bilddatensatzes (D_{v,non}) eines Patienten ladbar ist, mit Programmabschnitten, um alle Schritte des Verfahrens nach einem der Ansprüche 1 bis 11 auszuführen, wenn die Programmabschnitte von der Vorrichtung (45) ausgeführt werden.

15. Computerlesbares Speichermedium, auf welchem von einer Vorrichtung (45) zur Bereitstellung eines virtuellen, nicht-kontrastierten Bilddatensatzes (D_{v,non}) eines Patienten lesbare und ausführbare Programmabschnitte gespeichert sind, um alle Schritte des Verfahrens nach einem der Ansprüche 1 bis 11 auszuführen, wenn die Programmabschnitte von der Vorrichtung (45) ausgeführt werden.

## Claims

1. Method for providing a virtual, noncontrast image dataset (D_{v,non}) of a patient (39) comprising the steps
- provision (S1) of a multiphase CT angiography image dataset of the patient (39) comprising at least three CT image datasets (D₁, D₂, D₃) that map an imaging area of the patient at three different points in time relative to an administration of contrast agent,
**characterised by**:
- formation (S2) of a minimum intensity image dataset (Dₘᵢₙ) of the imaging area on the basis of the at least three CT image datasets (D₁, D₂, D₃), wherein the image value of an image point of the minimum intensity image dataset (Dₘᵢₙ) is in each case based on the minimum value from among the image values of the image points, locally corresponding in the imaging area, in the at least three CT image datasets (D₁, D₂, D₃), wherein the minimum value corresponds to the value that corresponds to the highest transmission of X-ray radiation, and thus the least absorption,
- output (S3) of the virtual, noncontrast image dataset (D_{v,non}) on the basis of the minimum intensity image dataset (Dₘᵢₙ), wherein the virtual, noncontrast CT image dataset (D_{v,non}) is generated by means of a computing unit on the basis of measured data that has been captured with the administration of contrast agent.

2. Method according to claim 1, wherein the relative time interval between the first CT image dataset in time (D₁) and the second CT image dataset (D₂) of the at least three CT image datasets (D₁, D₂, D₃) or the relative time interval between the second CT image dataset in time (D₂) and the third CT image dataset (D₃) of the at least three CT image datasets (D₁, D₂, D₃) is at least 5 s.

3. Method according to one of the preceding claims, wherein the imaging area comprises the brain of the patient (39).

4. Method according to one of the preceding claims, wherein an acquisition area of the first CT image dataset (D₁) of the at least three CT image datasets (D₁, D₂, D₃) differs from an acquisition area of the second CT image dataset (D₂) and/or third CT image dataset (D₃) of the at least three CT image datasets (D₁, D₂, D₃).

5. Method according to claim 4, wherein the acquisition area of the first CT image dataset (D₁) at least comprises the patient (39) from the aortic arch to the crown, and wherein the acquisition area of the second CT image dataset (D₂) and/or the acquisition area of the third CT image dataset (D₃) at least maps the patient (39) from the base of the skull to the crown.

6. Method according to one of the preceding claims, also comprising the step:
- performance (S4) of a motion correction of the at least three CT image datasets (D₁, D₂, D₃), as a result of which motion-corrected CT image datasets (M₁, M₂, M₃) are provided, and
wherein the minimum intensity image dataset (Dₘᵢₙ) is formed on the basis of the motion-corrected CT image datasets (M₁, M₂, M₃).

7. Method according to one of the preceding claims, also comprising the steps
- provision (S5) of a native, noncontrast CT image dataset (D_{n,non}) that maps an imaging area of the patient prior to administration of contrast agent, and
- correction (S6) of the minimum intensity image dataset (Dₘᵢₙ) using the native, noncontrast CT image dataset (D_{n,non}), as a result of which a corrected minimum intensity image dataset (D_{min,corr}) is provided, and
wherein
the virtual, noncontrast image dataset (D_{v,non}) that is output is based on the corrected minimum intensity image dataset (D_{min,corr}).

8. Method according to claim 7, wherein the step of correction (S6) comprises ascertaining image areas in the minimum intensity image dataset (Dₘᵢₙ) or at least one of the at least three CT image datasets, that exceed a particular noise level value or that fall below a particular intensity value, and ascertaining a correction value on the basis of a comparison between image areas in each case locally corresponding thereto in the minimum intensity image dataset (Dₘᵢₙ) and in the native, noncontrast CT image dataset (D_{n,non}).

9. Method according to claim 7, wherein the step of correction (S6) comprises creating a perfusion image dataset on the basis of the at least three CT image datasets (D₁, D₂, D₃) of the multiphase CT angiography image dataset of the patient (39), identifying image areas of the perfusion image dataset with locally increased contrast agent absorption, and ascertaining a correction value on the basis of a comparison between image areas locally corresponding thereto in the minimum intensity image dataset (Dₘᵢₙ) and locally corresponding image areas in the native, noncontrast CT image dataset (D_{n,non}).

10. Method according to one of claims 8 or 9, wherein the correction value is based on a ratio of the image values in the determined image areas of the minimum intensity image dataset (Dₘᵢₙ) or of the native, noncontrast CT image dataset (D_{n,non}) to image values in a surrounding area of the determined image areas of the minimum intensity image dataset (Dₘᵢₙ) or of the native, noncontrast CT image dataset (D_{n,non}).

11. Method for providing a perfusion image dataset, wherein
- a multiphase CT angiography image dataset of the patient (39) comprising at least three CT image datasets (D₁, D₂, D₃) that map an imaging area of the patient at three different points in time relative to an administration of contrast agent is provided,
- according to one of the preceding claims a virtual, noncontrast CT image dataset (D_{v,non}) of the imaging area is provided on the basis of the at least three CT image datasets (D₁, D₂, D₃), and
- a perfusion image dataset is generated and provided on the basis of the at least three CT image datasets (D₁, D₂, D₃) and the virtual, noncontrast CT image dataset (D_{v,non}).

12. Apparatus (45) for providing a virtual, noncontrast image dataset (D_{v,non}) of a patient comprising
- a first interface (IF1), designed to provide at least one multiphase CT image dataset of the patient, comprising at least three CT image datasets (D₁, D₂, D₃), which in each case map an imaging area of the patient at three different points in time relative to an administration of contrast agent,
- a computing unit (CU), designed to form a minimum intensity image dataset (Dₘᵢₙ) of the imaging area on the basis of the at least three CT image datasets (D₁, D₂, D₃), wherein the image value of an image point of the minimum intensity image dataset (Dₘᵢₙ) is in each case based on the minimum value from among the image values of the image points, locally corresponding in the imaging area, in the at least three CT image datasets (D₁, D₂, D₃), wherein the minimum value corresponds to the value that corresponds to the highest transmission of X-ray radiation, and thus the least absorption, and
- a second interface (IF2), designed to output the virtual noncontrast image dataset (D_{v,non}) on the basis of the minimum intensity image dataset (Dₘᵢₙ), wherein the virtual, noncontrast CT image dataset (D_{v,non}) is generated by means of a computing unit on the basis of measured data that has been captured with the administration of contrast agent.

13. Computed tomography device (32) having an apparatus (45) according to claim 12.

14. Computer program product having a computer program which can be loaded directly into a memory (MU) of an apparatus (45) for providing a virtual, noncontrast image dataset (D_{v,non}) of a patient, with program sections in order to execute all steps of the method according to one of claims 1 to 11, if the program sections are executed by the apparatus (45).

15. Computer-readable storage medium, on which are stored program sections which can be read and executed by an apparatus (45) for providing a virtual, noncontrast image dataset (D_{v,non}) of a patient, in order to execute all steps of the method according to one of claims 1 to 11, if the program sections are executed by the apparatus (45).

## Revendications

1. Procédé pour disposer d'un ensemble (D_{v,non}) virtuel non contrasté de données d'image d'un patient (39) comprenant les stades
- se procurer (S1) un ensemble de données d'image d'angiographie par tomodensitométrie assistée par ordinateur à plusieurs phases du patient (39), comprenant au moins trois ensembles (D₁, D₂, D₃) de données d'image par tomodensitométrie assistée par ordinateur, qui représentent une partie représentée du patient à trois instants différents par rapport à une dose d'agent de contraste,
**caractérisé par** :
- formation (S2) d'un ensemble (Dₘᵢₙ) de données d'image d'intensité minimum de la partie représentée, sur la base des au moins trois ensembles (D₁, D₂, D₃) de données d'image par tomodensitométrie assistée par ordinateur, dans lequel la valeur d'image d'un point image de l'ensemble (Dₘᵢₙ) de données d'image d'intensité minimum repose respectivement sur la valeur minimum parmi les valeurs d'image des points image correspondants localement dans la partie représentée dans les au moins trois ensembles (D₁, D₂, D₃) de données d'image par tomodensitométrie assistée par ordinateur, dans lequel la valeur minimum correspond à la valeur, qui correspond à la transmission la plus grande du rayonnement X et ainsi à l'absorption la plus petite,
- sortie (S3) de l'ensemble (D_{v,non}) de données d'image virtuel non contrasté sur la base de l'ensemble (Dₘᵢₙ) de données d'image d'intensité minimum, dans lequel l'ensemble (D_{v,non}) de données d'image par tomodensitométrie assistée par ordinateur virtuel non contrasté est produit au moyen d'une unité informatique reposant sur des données de mesure, qui ont été saisies avec une dose d'agent de contraste.

2. Procédé suivant la revendication 1, dans lequel le laps de temps relatif entre le premier chronologiquement ensemble (D₁) de données d'image par tomodensitométrie assistée par ordinateur et le deuxième ensemble (D₂) de données d'image par tomodensitométrie assistée par ordinateur des au moins trois ensembles (D₁, D₂, D₃) de données d'image par tomodensitométrie assistée par ordinateur, ou le laps de temps relatif entre le deuxième chronologiquement ensemble (D₂) de données d'image par tomodensitométrie assistée par ordinateur et le troisième ensemble (D₃) de données d'image assisté par ordinateur des au moins trois ensembles (D₁, D₂, D₃) de données d'image par tomodensitométrie assistée par ordinateur, est d'au moins 5 secondes.

3. Procédé suivant l'une des revendications précédentes, dans lequel la partie représentée comprend le cerveau du patient (39).

4. Procédé suivant l'une des revendications précédentes, dans lequel une partie d'enregistrement du premier ensemble (D₁) de données d'image par tomodensitométrie assistée par ordinateur des au moins trois ensembles (D₁, D₂, D₃) de données d'image par tomodensitométrie assistée par ordinateur, se distingue d'une partie d'enregistrement du deuxième ensemble (D₂) de données d'image par tomodensitométrie assistée par ordinateur et/ou du troisième ensemble (D₃) de données d'image par tomodensitométrie assistée par ordinateur des au moins trois ensembles (D₁, D₂, D₃) de données d'image par tomodensitométrie assistée par ordinateur.

5. Procédé suivant la revendication 4, dans lequel la partie d'enregistrement du premier ensemble (D₁) de données d'image par tomodensitométrie assistée par ordinateur comprend au moins le patient (39) de la crosse de l'aorte au vertex, et dans lequel la partie d'enregistrement du deuxième ensemble (D₂) de données d'image par tomodensitométrie assistée par ordinateur et/ou la partie d'enregistrement du troisième ensemble (D₃) de données d'image par tomodensitométrie assistée par ordinateur, représente au moins le patient (39) de la base du crâne au vertex.

6. Procédé suivant l'une des revendications précédentes, comprenant en outre le stade :
- effectuer (S4) une correction de mouvement des au moins trois ensembles (D₁, D₂, D₃) de données d'image par tomodensitométrie assistée par ordinateur, grâce à quoi, des ensembles (M₁, M₂, M₃) corrigés de données d'image par tomodensitométrie assistée par ordinateur sont mis à disposition, et
dans lequel l'ensemble (Dₘᵢₙ) de données d'image d'intensité minimum est formé sur la base des ensembles (M₁, M₂, M₃) corrigés en mouvement des données d'image par tomodensitométrie assistée par ordinateur.

7. Procédé suivant l'une des revendications précédentes, comprenant en outre les stades
- se procurer (S5) un ensemble (D_{n, non}) naturel non contrasté de données d'image par tomodensitométrie assistée par ordinateur, qui représente une partie représentée du patient avant la dose d'agent de contraste, et
- corriger (S6) l'ensemble (Dₘᵢₙ) de données d'image d'intensité minimum en utilisant l'ensemble (D_{n, non}) naturel non contrasté de données d'image par tomodensitométrie assistée par ordinateur, grâce à quoi un ensemble (D_{min, korr}) corrigé de données d'image d'intensité minimum est mis à disposition, et dans lequel
l'ensemble (D_{v, non}) virtuel sorti non contrasté de données d'image repose sur l'ensemble (D_{min, korr}) corrigé de données d'image d'intensité minimum.

8. Procédé suivant la revendication 7, dans lequel le stade de la correction (S6) comprend déterminer des parties d'image dans l'ensemble (Dₘᵢₙ) de données d'image d'intensité minimum ou du moins dans l'un des au moins trois ensembles de données d'image par tomodensitométrie assistée par ordinateur, qui dépassent une valeur de bruit déterminée ou qui sont inférieures à une valeur d'intensité déterminée et, sur la base d'une comparaison entre respectivement des parties d'image correspondantes localement dans l'ensemble (Dₘᵢₙ) de données d'image d'intensité minimum et dans l'ensemble (D_{n, non}) naturel non contrasté de données d'image par tomodensitométrie assistée par ordinateur, déterminer une valeur de correction.

9. Procédé suivant la revendication 7, dans lequel le stade de la correction (S6) comprend, sur la base des au moins trois ensembles (D₁, D₂, D₃) de données d'image par tomodensitométrie assistée par ordinateur, de l'ensemble de données d'image d'angiographie par tomodensitométrie assistée par ordinateur à plusieurs phases du patient (39), établir un ensemble de données d'image de perfusion, identifier des parties d'image de l'ensemble de données d'image de perfusion ayant un enregistrement d'agent de contraste augmenté localement et, sur la base d'une comparaison entre des parties d'image correspondantes localement dans l'ensemble (Dₘᵢₙ) de données d'image d'intensité minimum et des parties d'image correspondantes localement dans l'ensemble (D_{n, non}) naturel non contrasté de données d'image par tomodensitométrie assistée par ordinateur, déterminer une valeur de correction.

10. Procédé suivant l'une des revendications 8 ou 9, dans lequel la valeur de correction repose sur un rapport des valeurs d'image dans les parties d'image déterminées de l'ensemble (Dₘᵢₙ) de données d'image d'intensité minimum ou de l'ensemble (D_{n, non}) naturel non contrasté de données d'image par tomodensitométrie assistée par ordinateur à des valeurs d'image dans un environnement des parties d'image déterminées de l'ensemble (Dₘᵢₙ) de données d'image d'intensité minimum ou de l'ensemble (D_{n, non}) naturel non contrasté de données d'image par tomodensitométrie assistée par ordinateur.

11. Procédé pour disposer d'un ensemble de données d'image de perfusion, dans lequel
- on se procure un ensemble de données d'image d'angiographie par tomodensitométrie assistée par ordinateur à plusieurs phases du patient (39), comprenant au moins trois ensembles (D₁, D₂, D₃) de données d'image par tomodensitométrie assistée par ordinateur, qui représentent une partie représentée du patient à trois instants différents relativement à une dose d'agent de contraste,
- suivant l'une des revendications précédentes, en se fondant sur les au moins trois ensembles (D₁, D₂, D₃) de données d'image par tomodensitométrie assistée par ordinateur, on se procure un ensemble (D_{v, non}) virtuel non contrasté de données d'image par tomodensitométrie assistée par ordinateur de la partie représentée, et
- sur la base des au moins trois ensembles (D₁, D₂, D₃) de données d'image par tomodensitométrie assistée par ordinateur et de l'ensemble (D_{v, non}) virtuel non contrasté de données d'image par tomodensitométrie assistée par ordinateur, on produit et met à disposition un ensemble de données d'image de perfusion.

12. Dispositif (45) pour disposer d'un ensemble (D_{v, non}) virtuel non contrasté de données d'image d'un patient, comprenant
- une première interface (IF1), constituée pour disposer d'au moins un ensemble de données d'image par tomodensitométrie assistée par ordinateur à plusieurs phases du patient, comprenant au moins trois ensembles (D₁, D₂, D₃) de données d'image par tomodensitométrie assistée par ordinateur, qui représentent chacun une partie représentée du patient à trois instants différents par rapport à une dose d'agent de contraste,
- une unité (CU) informatique, constituée pour former un ensemble (Dₘᵢₙ) de données d'image d'intensité minimum de la partie représentée sur la base des au moins trois ensembles (D₁, D₂, D₃) de données d'image par tomodensitométrie assistée par ordinateur, dans lequel la valeur d'image d'un point image de l'ensemble (Dₘᵢₙ) de données d'image d'intensité minimum repose respectivement sur la valeur minimum parmi les valeurs d'image des points image correspondants localement dans la partie représentée dans les au moins trois ensembles (D₁, D₂, D₃) de données d'image par tomodensitométrie assistée par ordinateur, dans lequel la valeur minimum correspond à la valeur, qui correspond à la transmission la plus grande du rayonnement X et ainsi à l'absorption la plus petite, et
- une deuxième interface (IF2), constituée pour sortir l'ensemble (D_{v, non}) virtuel non contrasté de données d'image sur la base de l'ensemble (Dₘᵢₙ) de données d'image d'intensité minimum, dans lequel l'ensemble (D_{v, non}) virtuel non contrasté de données d'image par tomodensitométrie assistée par ordinateur a été produit au moyen d'une unité informatique sur la base de données de mesure, qui ont été saisies avec une dose d'agent de contraste.

13. Appareil (32) de tomodensitométrie assisté par ordinateur, comportant un dispositif (45) suivant la revendication 12.

14. Produit de programme d'ordinateur comprenant un programme d'ordinateur, qui peut être chargé directement dans la mémoire (MU) d'un dispositif (45) pour disposer d'un ensemble (D_{v, non}) virtuel non contrasté de données d'image d'un patient, comprenant des parties de programme pour exécuter tous les stades du procédé suivant l'une des revendications 1 à 11, lorsque des parties de programme sont exécutées par le dispositif (45).

15. Support de mémoire déchiffrable par ordinateur, sur lequel sont mises en mémoire des parties de programme pouvant être lues et réalisées par un dispositif (45) de mise à disposition d'un ensemble (D_{v, non}) virtuel non contrasté de données d'image d'un patient, afin d'exécuter tous les stades du procédé suivant l'une des revendications 1 à 11, lorsque les parties de programme sont exécutées par le dispositif (45).
